# FASCICULE DE BREVET EUROPEEN

(11) **EP 0 419 621 B1**
(45) Date de publication et mention de la délivrance du brevet: **27.12.1995**
(21) Numéro de dépôt: 90905207.8
(22) Date de dépôt: 19.03.1990
(51) Int. Cl.: C12N 15/00, C12N 15/85, C12N 15/90, G01N 33/68

(54) **PROCEDE DE REMPLACEMENT SPECIFIQUE D'UNE COPIE D'UN GENE PRESENT DANS LE GENOME RECEVEUR PAR L'INTEGRATION D'UN GENE DIFFERENT DE CELUI OU SE FAIT L'INTEGRATION**
VERFAHREN ZUM SPEZIFISCHEN ERSETZEN EINER GENKOPIE, DIE SICH IN EINEM REZEPTORENGENOM BEFINDET, DURCH GENINTEGRATION
PROCESS FOR THE SPECIFIC REPLACEMENT OF A COPY OF A GENE PRESENT IN THE RECEIVER GENOME VIA THE INTEGRATION OF A GENE

(30) Priorité: 20.03.1989 FR 8903630
(43) Date de publication de la demande: 03.04.1991
(60) Demande divisionnaire: 95107951.6
(73) Titulaire: INSTITUT PASTEUR, 75724 Paris Cédex 15 (FR)
(72) Inventeur: LE MOUELLIC, Hervé, F-75015 Paris (FR); BRULLET, Philippe, F-78310 Maurepas (FR)
(74) Mandataire: Gutmann, Ernest
(86) Numéro de dépôt international: FR9000185
(87) Numéro de publication internationale: WO9011354

(56) Documents cités:
- EP-A- 0 074 808
- EP-A- 0 169 672
- EP-A- 0 279 582
- EP-A- 0 289 121
- Cell, Vol. 56, 27 Janvier 1989, Cell Press, (Cambridge, Mass., US), S. THOMPSON et al.: "Germ Line Transmission and Expression of a Corrected HPRT Gene Produced by Gene Targeting in Embryonic Stem Cells", pages 313-321
- Nature, Vol. 338, 9 Mars 1989, (Londres, GB), A. ZIMMER et al.: "Production of Chimaeric Mice Containing Embryonic Stem (ES) Cells Carring a Homoeobox Hox 1.1 Allele Mutated by Homologous Recombination", pages 150-153
- Nature, Vol. 336, 24 Novembre 1988, (Londres, US), S.L. MANSOUR et al.: "Disruption of the Proto-Oncogene Int-2 in mouse Embryo-Derived Stemm Cells: A General Strategy for Targeting Mutations to Non-Selectable Genes", pages 348- 351
- Proc. Natl. Acad. Sci. USA, Vol. 85, Novembre 1988, (Washington, US), T. DOETSCHMAN et al.: "Targeted Mutation of the Hprt Gene in mouse Embryonic Stemm Cells", pages 8583-8587
- CHEMICAL ABSTRACTS, Vol. 110, No. 17, 24 Avril 1989, (Columbus, Ohio US), M.A. FROHMAN et al.: voir page 194, rèsumè 148796z, & Cell
- CHEMICAL ABSTRACTS, Vol. 111, No. 21, 20 Novembre 1989, (Columbus, Ohio, US), R.S. JOHNSON et al.: "Targeting of Non-Expressed Genes in Embryonic Stem Cells Via Homologous Recombination", voir page 205* Resume 188649f, & Science (Washington, D.C., 1883-), 1989, 245(4923), 1234-6*

## Description

L'invention concerne un procédé de remplacement spécifique d'une copie d'un gène présent dans le génome d'un organisme eucaryote receveur par l'intégration d'un gène différent du gène inactivé. De préférence, le gène receveur sera présent en au moins 2 exemplaires dans la cellule hôte transfectée. Le gène receveur est défini comme étant le gène ou se fera l'insertion du gène différent.

Plus particulièrement, l'invention concerne la production d'animaux transgéniques dans lesquels le gène étranger a été introduit d'une manière ciblée pour permettre, à la fois, le maintien des fonctions génétiques normales de l'animal et l'expression du gène étranger sous le contrôle de promoteurs endogènes.

Par "gène différent ou étranger" on entend toute séquence nucléotidique correspondant à la totalité ou à une partie d'un gène "étranger ou différent" du gène récepteur telle qu'elle est trouvée normalement dans le génome (ARN ou ADN), ou elle correspond également à une séquence modifiée artificiellement du gène normal ou encore à un fragment de cette séquence.

L'invention concerne également le procédé de production de ces animaux transgéniques.

Dans la production d'animaux transgéniques, les méthodes conventionnelles utilisées pour l'introduction de séquences d'ADN hétérologues dans la lignée cellulaire germinale, ne permettent pas de contrôler le site de l'intégration du gène étranger dans le génome, ni le nombre de copies ainsi introduit. L'intégration du gène étranger se fait au hasard et, en général, plusieurs copies du gène s'intègrent en même temps, parfois sous forme de tandem tête à queue, le site de l'intégration et le nombre de copies intégrées variant d'un animal transgénique à l'autre.

Il peut donc arriver que des gènes cellulaires endogènes, situés au point d'insertion, soient ainsi inactivés, sans que cela soit facilement décelable en raison de nombreuses insertions au hasard. Si le produit de ces gènes est important pour le développement de l'animal, celui-ci sera sérieusement perturbé. D'ailleurs, l'insertion aléatoire du gène étranger peut se faire à un site qui n'est pas approprié pour l'expression du gène. De plus, le fait qu'il y ait variation du site et du nombre d'insertions d'animal en animal rend l'interpétation des études d'expression extrêmement difficile.

Un problème majeur rencontré dans la production d'animaux transgéniques, est l'obtention de l'expression du gène étranger. D'une manière générale, deux types d'expérience ont été réalisés chez les souris.

Les gènes introduits dans la lignée germinale sont :
- soit des gènes "complets", comprenant des séquences codantes flanquées de leurs propres séquences régulatrices;
- soit des gènes composites, formés de la séquence codante d'un gène fusionnée à la séquence promotrice d'un autre gène, les deux fragments appartenant même parfois à deux espèces animales différentes.

On a pu ainsi confirmer que la spécificité de l'expression des gènes dans tel ou tel tissu est déterminée par leur(s) séquence(s) régulatrices.

Le choix du promoteur approprié pour l'expression du gène étranger chez l'animal transgénique est donc d'une importance primordiale.

D'autre part, la mutagénèse dirigée de gènes murins dans des cellules souches embryonnaires a récemment été réalisée en faisant appel à une technique de "ciblage génétique" (gene targeting) (Thomas et al., 1987; Thompson et al., 1989).

Dans le premier cas, le gène murin HPRT a été muté par insertion et remplacement et, dans le deuxième cas, un gène HPRT muté a été corrigé. Thomson et al. ont étendu leurs expériences jusqu'à l'obtention de souris chimères et ont constaté le passage de la modification génétique dans la lignée cellulaire germinale.

Dans chacun des documents cités, le site précis de l'intégration a été ciblé par recombinaison homologue entre, d'une part, des séquences exogènes comportant la mutation ou correction incluses dans un vecteur, sous le contrôle d'un promoteur exogène, et, d'autre part, leur homologue génomique. Ceci étant, il faut remarquer que les auteurs antérieurs ont réalisé leurs expériences sur un gène spécifique (HPRT) dont l'activation par mutation s'accompagnait d'un phénotype décelable. La mutation ciblée décrite par Thomas et al., avait pour effet d'inactiver le gène HPRT et, par conséquent, de faire disparaître le phénotype décelable normalement associé avec le HPRT. Le gène de sélection Neo^{R}, sous le contrôle d'un promoteur TK, était donc incorporé dans l'ADN d'insertion afin de permettre la sélection des transformants. Il est à noter que les expériences décrites dans l'art antérieur impliquaient une sélection soit par le gène receveur (p. ex HPRT) soit par le gène d'insertion (p. ex Neo^{R}). Le site de l'insertion et/ou le type de gène inséré est donc limité à des gènes conférant un caractère sélectable.

En outre, dans l'art antérieur, les séquences exogènes sur le vecteur servent donc à la fois à cibler le site d'intégration et à introduire la modification. Suite à la recombinaison homologue, le gène modifié se trouve toujours dans son environnement génétique normal.

Rappelons qu'un problème qui se pose au cours de la production d'animaux transgéniques est le danger d'inactiver un gène cellulaire endogène qui se trouve au point d'insertion du gène étranger.

Selon la fonction du produit du gène inactivé, une telle inactivation peut conduire à des désordres physiologiques ou morphologiques importants chez l'animal transgénique, ou pourrait même empêcher sa survie.

En revanche, l'inactivation d'un gène pourrait être considéré comme avantageux si le gène en question codait pour un récepteur de virus ou autre agent infectieux.

Les inventeurs ont étudié la possibilité d'éviter les inconvénients décrits plus haut, et associés, dans certains cas, à l'inactivation possible d'un ou plusieurs gènes cellulaires endogène de fonction importante au cours de la production d'animaux transgéniques.

L'invention a pour objet un procédé de remplacement spécifique, notamment par ciblage d'un ADN, dit ADN d'insertion constitué par une partie d'un gène susceptible d'être rendu fonctionnel, ou dont le fonctionnement peut être rendu plus efficace, lorsqu'il est recombiné avec un ADN de complément pour alors fournir un gène recombinant complet dans le génome d'une cellule eucaryote, caractérisé en ce que
- le site d'insertion se trouve dans un gène choisi, dit gène receveur, et contenant l'ADN de complément, et en ce que
- l'on transfecte des cellules eucaryotes avec un vecteur contenant un insert comprenant lui-même l'ADN d'insertion et deux séquences dites "flanquantes" de part et d'autre de l'ADN d'insertion, respectivement homologues à deux séquences génomiques qui jouxtent le site d'insertion souhaité dans le gène receveur,
- l'ADN d'insertion étant hétérologue vis-à-vis du gène receveur, et
- les séquences flanquantes, étant choisies parmi celles qui constituent le susdit ADN de complément et qui autorisent, par recombinaison homologue avec des séquences correspondantes du gène receveur, la reconstitution d'un gène recombinant complet dans le génome de la cellule eucaryote à condition que lorsque l'ADN d'insertion est constitué d'une séquence codante, il ne s'agit pas de la séquence codante d'un gène codant pour un agent de sélection.

L'invention concerne aussi un procédé de production d' animaux transgéniques, caractérisé en ce que des cellules E.S. sont transfectées dans les conditions sus-décrites et sélectionnées pour l'évènement de recombinaison homologue, à savoir l'intégration correcte du gène étranger, les cellules transfectées sont injectées dans des embryons à un stade où ils sont aptes à intégrer les cellules transfectées (par exemple au stade blastocyste), ceux-ci sont ensuite réimplantés dans une mère porteuse et les individus chimères obtenus au terme de la gestation sont accouplés. Si les cellules E.S. ont colonisé la lignée germinale de l'animal chimère, des animaux transgéniques hétérogozytes pour le gène remplacé seront obtenus par accouplement (F1) dans la descendance.

Il est également possible d'insérer le gène, porté par le vecteur de l'invention, dans l'oeuf peu de temps après la fécondation (c'est-à-dire moins de 24 heures). De cette manière, l'insertion est effectuée pendant que l'oeuf est à l'état unicellulaire.

L'invention concerne aussi un plasmide apte à effectuer l'insertion ciblée d'un gène recombinant dit gène d'insertion dans le génome d'une cellule eucaryote, caractérisé en ce qu'il contient un insérat comprenant lui-même le gène d'insertion et deux séquences dites "flanquantes" de part et d'autre du gène d'insertion, respectivement homologues aux deux séquences génomiques qui jouxtent le site d'insertion souhaité dans le gène receveur.

Le procédé de l'invention permet, donc, grâce au phénomène de recombinaison homologue, d'insérer d'une manière ciblée des gènes étrangers , en particulier des séquences codantes dépourvues du promoteur qui leur est normalement associé, dans le génome d'un organisme eucaryote à un site qui permet leur expression sous le contrôle du promoteur endogène du gène où se fait l'insertion,' et par conséquence, d'inactiver le gène endogène ciblé.

Selon un mode de réalisation préféré de l'invention, le gène receveur ciblé est un gène qui est présent dans le génome en au moins deux exemplaires. L'utilisation de la technique d'électroporation (Ref. 11) assure l'introduction d'une copie seulement du gène étranger.

Selon cette variante de l'invention, l'insertion ciblée du gène d'intérêt (c'est-à-dire le gène dit d'insertion) a pour effet d'inactiver la seule copie du gène cellulaire endogène où se fait l'insertion et laisse intacte et fonctionnelle la ou les autre(s) copie(s) de ce gène.

De cette façon, le fonctionnement génétique de l'animal transgénique n'est pas ou peu perturbé par l'introduction du gène étranger, même si l'insertion inactive une seule copie d'un gène essentiel receveur pour le développement de l'animal. Soit son développement ne serait donc pas affecté par l'insertion du gène étranger, soit les perturbations mineures possibles dans le cas de l'inactivation d'un gène critique ne seraient probablement pas léthales pour l'animal. Les effets de l'insertion du gène étranger à l'état homozygote pourraient être de toute nature et seraient observés en 2ème génération (F2) après croisements d'individus hétérozygotes (F1) entre eux.

Si, par contre, l'inactivation de toutes les copies d'un gène est souhaitée, par exemple, dans le cas où le gène code pour un récepteur d'agent infectieux, de multiple copies du gène étranger sont introduites. Le contrôle de la quantité introduite peut être assuré en faisant appel à des techniques connues.

L'insertion ciblée du gène étranger permet donc son introduction dans un site où son expression est sous le contrôle des séquences régulatrices du gène endogène où se fait l'insertion.

Le procédé de l'invention permet ainsi d'insérer le gène étranger derrière un promoteur endogène qui a les fonctions désirées (par exemple, spécificité d'expression dans tel ou tel tissu), et cela, le cas échéant, sans inactiver les autres copies du gène receveur.
Selon un mode de réalisation particulièrement préféré de l'invention, l'ADN d'insertion comporte entre les séquences flanquantes, d'une part une séquence d'ADN destinée à être recombinée avec l'ADN de complément dans le gène receveur pour fournir un gène recombinant, et, d'autre part, une séquence codant pour un agent sélectif permettant la sélection des transformants et un promoteur autorisant l'expression de l'agent sélectif, le gène receveur et le gène recombinant codant pour des produits d'expression ne conférant pas de phénotype sélectionable.

De cette manière, la sélection des transformants est entièrement indépendante de la nature du gène receveur et du gène inséré, contrairement aux procédés décrits jusqu'à ce jour dans lesquels le gène inséré ou le gène receveur devait par nécessité coder pour un produit d'expression permettant la sélection des transformants. Le système développé par les inventeurs permet une flexibilité totale en ce qui concerne la nature du gène receveur et du gène inséré ou du gène formé par la recombinaison homologue. Les inventeurs ont constaté d'une manière surprenante que l'insertion de séquences de taille importante (par exemple d'environ 7.5 kb) n'affecte pas la fréquence de recombinaison homologue.
L'effet que peut avoir l'insertion de la séquence d'ADN selon cet aspect de l'invention inclut, selon le type de séquence insérée par exemple le remplacement d'une séquence codante, le remplacement d'une séquence régulatrice, l'inactivation ou la réactivation d'un gène par mutation ou l'amélioration du taux d'expression d'un gène. Il est possible, selon l'invention, de remplacer une phase codante ou une partie d'une phase codante par une séquence hétérologue qui commence au codon d'initiation du gène remplacé afin que l'expression du gène inséré remplace entièrement l'expression du gène remplacé. Ceci évite la formation de protéines de fusion qui pourrait être indésirable chez un animal transgénique.

Selon ce mode de réalisation de l'invention, l'ADN d'insertion peut comporter entre les séquences flanquantes une séquence codante hétérologue dépourvue de promoteur, la séquence codante étant autre qu'un gène codant pour un agent de sélection. L'ADN d'insertion peut comporter en outre, en aval de la séquence codante et toujours entre les séquences flanquantes, un gène codant pour un agent de sélection, associé à un promoteur permettant son expression dans la cellule cible. De cette manière, la séquence codante hétérologue peut être insérée derrière un promoteur endogène qui a les propriétés souhaitées, par exemple une certaine spécificité d'expression, ou grille de transcription etc, la sélectabilité des cellules transformées étant entièrement indépendante de l'expression de la séquence codante hétérologue. Ce type de construction permet, par exemple, de sélectionner les transformants même si le gène remplacé par la séquence codante hétérologue n'est pas normalement exprimé dans les cellules cibles. Ceci est particulièrement important dans la production d'animaux transgéniques à partir de cellule E.S. ("Embryonic Stem Cells") puisqu'une proportion importante des gènes reste inactive jusqu'à un stade plus avancé du développement de l'animal. Le gène Hox-3.1 est un exemple de ce type de gène. D'autre part, si la séquence codante code pour une protéine facilement décelable, par exemple, le 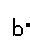 -Gal, le développement de la grille de transcription du gène endogène remplacé peut être suivi. Le vecteur pGN est un exemple de ce type de construction.

Selon un autre mode de réalisation de l'invention, l'ADN d'insertion peut comporter une séquence régulatrice étrangère. Le site d'insertion et, par conséquence, les séquences flanquantes sont choisies en fonction du but désiré, à savoir soit l'insertion de la séquence régulatrice étrangère pour donner un effet de "double promoteur" avec la séquence régulatrice endogène, ou soit le remplacement d'un promoteur endogène par le promoteur étranger. La séquence codante qui se trouve sous le contrôle de la séquence régulatrice peut être endogène.

Une autre possibilité serait l'insertion ciblée d'un ADN étranger qui comporte à la fois une séquence régulatrice et une séquence codante. Il est possible que la séquence régulatrice soit celle qui est naturellement associée avec la séquence codante.

Le procédé de l'invention met en oeuvre un vecteur contenant deux séquences "flanquantes" de part et d'autre du gène étranger. Ces séquences flanquantes ont au moins 150 paires de bases et sont de préférence inférieures à la longueur du gène receveur. Il est essentiel que ces deux séquences flanquantes soient homologues aux deux séquences génomiques qui jouxtent le site d'insertion souhaité. La séquence flanquante du vecteur qui se trouve en amont du gène étranger à introduire, est normalement homologue à la séquence génomique qui est située du côté 5' du site d'insertion. De la même manière, la séquence flanquante du vecteur qui se trouve en aval du gène étranger, est normalement homologue à la séquence génomique qui est située au côté 3' du site d'insertion.

Il est possible d'introduire des séquences "intercalantes" entre l'une ou l'autre des séquences flanquantes et le gène étranger, par exemple des séquences permettant la sélection des transformants, des marqueurs, des séquences permettant le clonage du vecteur, etc...

La position de ces séquences intercalantes vis-à-vis du gène étranger doit pourtant être choisie afin de ne pas empêcher l'expression du gène étranger, en particulier de la séquence d'ADN codante étrangère sous le contrôle du promoteur endogène ou, à l'inverse, la séquence codante d'ADN endogène sous le contrôle d'éléments de régulation étrangers apportés par la séquence d'insertion.

Malgré la présence des séquences flanquantes, qui encourage une recombinaison homologue, il est possible qu'un certain nombre d'intégrations se fasse au hasard. Afin de vérifier que l'insertion ciblée a bien eu lieu dans le site ciblé et non pas dans un autre endroit, la technique du "Polymerase Chain Reaction" (P.C.R.) (voir Ref. 10) est utilisée pour amplifier la séquence d'ADN du locus où l'insertion aurait dû se faire. De cette façon, seuls les clones transformés à la suite d'une recombinaison homologue sont sélectionnés.

Les séquences flanquantes du vecteur sont bien évidemment choisies en fonction du site d'insertion désiré pour que la recombinaison homologue puisse avoir lieu. Le cas échéant, les séquences flanquantes peuvent comporter des séquences répliques du promoteur endogène et/ou des modifications aux séquences qui précèdent le codon d'initiation pour améliorer le taux de traduction (séquences amont) et séquences répliques des séquences de terminaison notamment des sites de polyadénylation (séquences aval).

Le gène d'insertion peut être n'importe quel gène d'intérêt. On citera comme exemples non-limitatifs, le gène lac Z (comme dans le modèle décrit plus loin), les gènes codant pour l'interleukine ou l'interféron, les gènes de récepteur, par exemple de l'acide rétinoique ou beta-3 adrénergique ou de H.I.V., et des gènes connus comme étant liés à certaines maladies, par exemple la myopathie, etc...

Selon une variante préférée de l'invention, les cellules eucaryotes sont des cellules souches embryonnaires (voir Ref. 14 et 15).

En effet, une cellule E.S. mutée peut être injectée dans un embryon précoce qui, après réimplantation, pourra naître sous une forme chimère. Si la lignée germinale est colonisée par la cellule mutée, l'animal chimère transmettra la mutation à sa descendance. Par la suite, on pourra observer les effets de cette mutation, à l'état homozygote chez certains individus, sur leur développement, leur comportement, leur métabolisme, leur pathologie, etc..

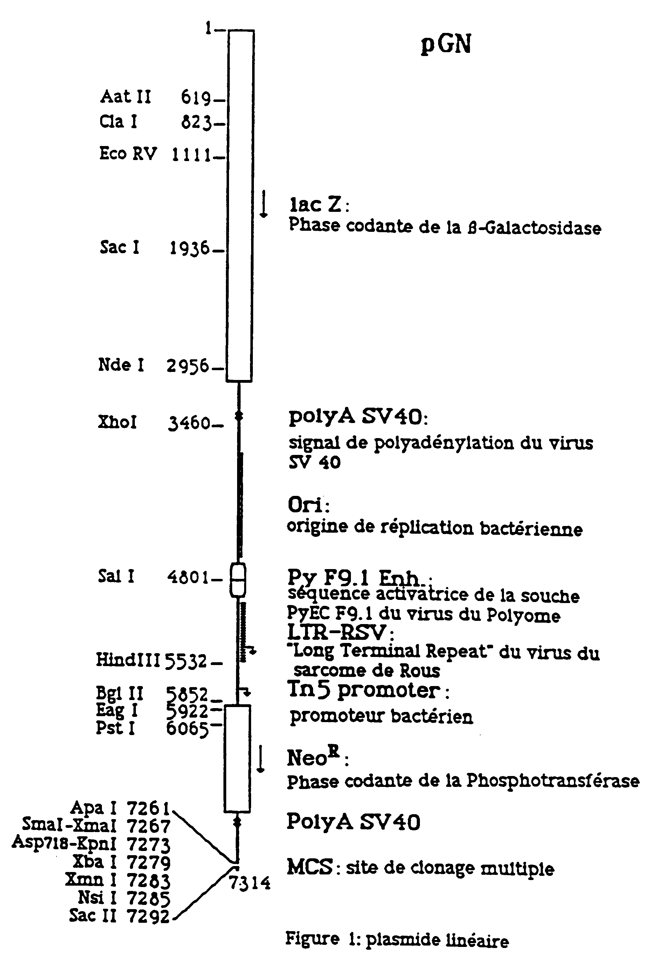

La figure 1 montre le plasmide pGN,
Les figures 2 a et b montrent les molécules pGMA et pGMD respectivement construites à partir du plasmide pGN par rapport au gène Hox-3.1. Ces plasmides sont des plasmides de mutagénèse. Les deux parties de la phase codante du gène Hox-3.1 sont représentées, sur le chromosome 15, avec la boîte "homéo" en noir. Les séquences correspondantes de Hox-3.1 ont été clonées dans le plasmide pGN. (A : signal de polyadénylation; Enh/Pro : enhancer-promoteur).
07 et 08 figurent les deux oligonucléotides utilisés dans la PCR.

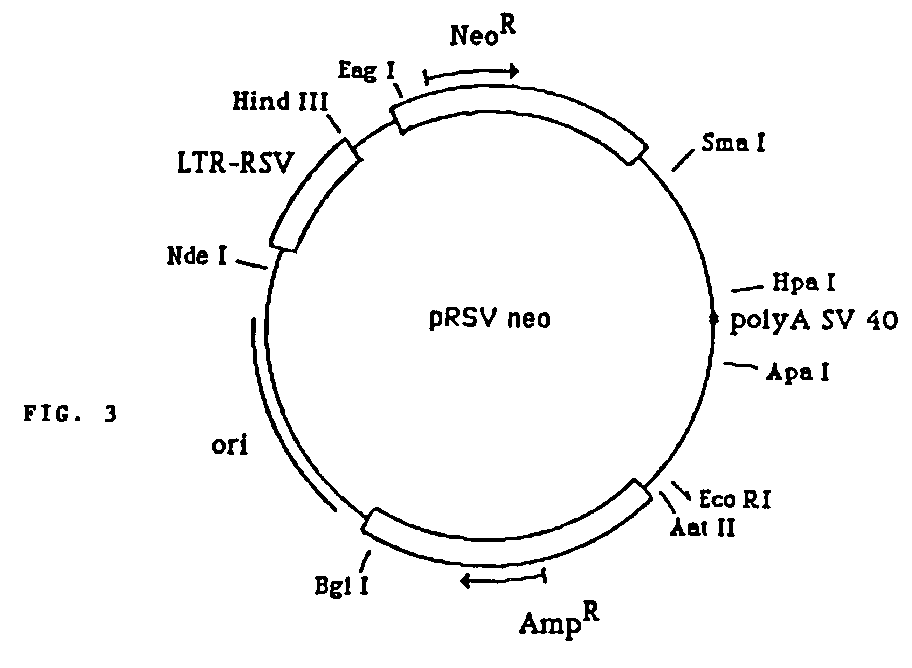

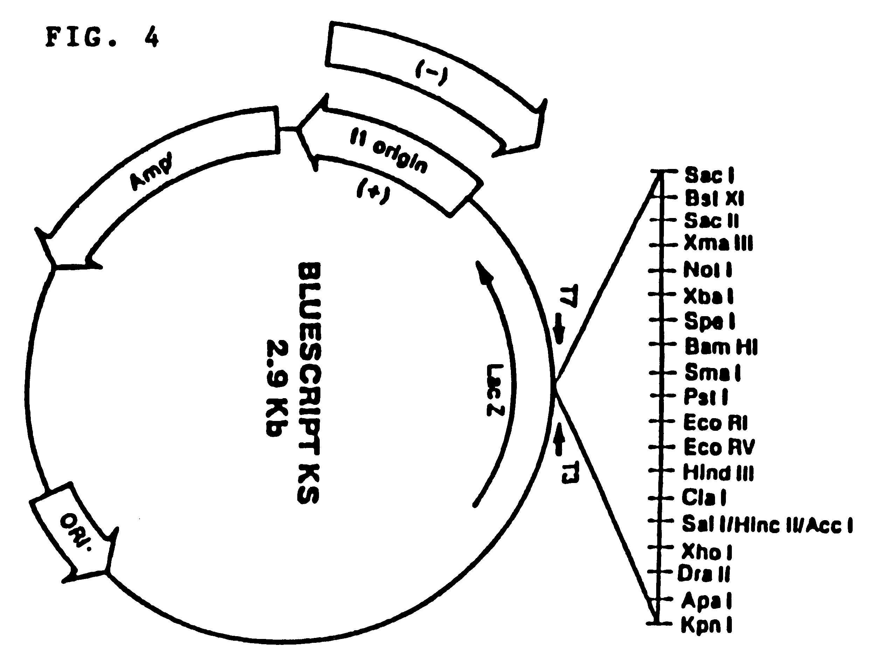

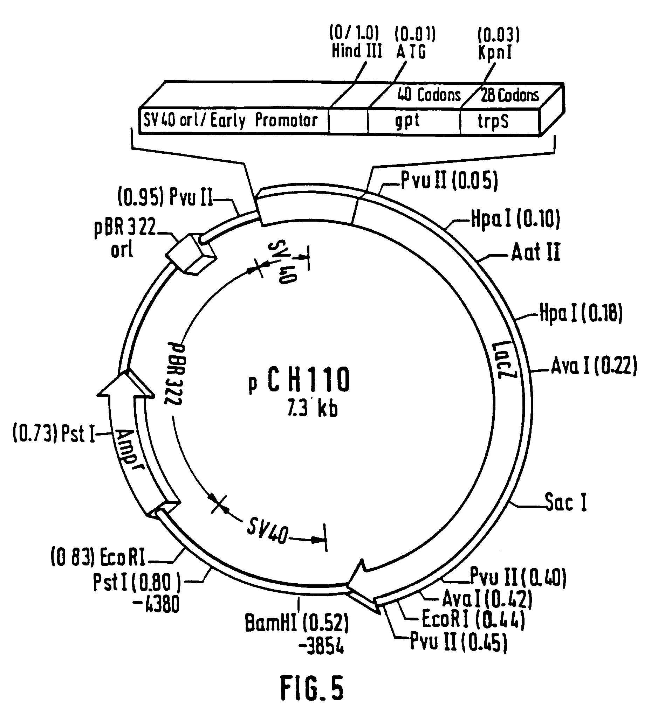

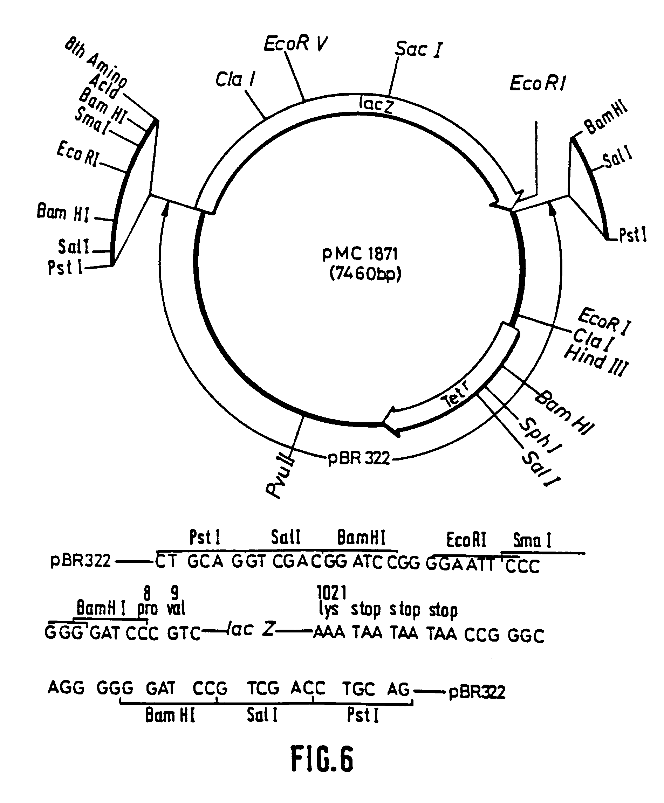

Les figures 3 à 6 montrent les plasmides utilisés dans la construction du pGN.

La figure 7 illustre la détection de recombinaison homologue avec la technique Réaction de Polymérase en Chaîne (P.C.R.) sur des cellules E.S. transfectées.

La figure 8 (a) et (b) montre des analyses de Southern de clones individus positifs (L5 et F2) et cellules E.S. (C.C.E.).

Le procédé de l'invention est d'application industrielle très large et peut varier selon la nature du gène étranger introduit.

La génétique des mammifères va progresser de manière considérable grâce à la possibilité récente de mutagéniser spécifiquement n'importe quel gène, permettant ainsi de mieux définir son rôle. Par cette technologie qui fait intervenir recombinaison homologue et cellules E.S., des informations précieuses seront apportées sur des oncogènes, des facteurs de croissance, des facteurs de transcription, etc..., gènes qui concernent des sujets très actuels de la recherche fondamentale ou la recherche appliquée. Un débouché important pour la recherche médicale est la possibilité de reproduire une maladie humaine dont la détermination génétique est connue (certaines maladies humaines à pathologie, telle la myopathie de Duchesne) ceci afin de mieux en étudier les mécanismes et de rechercher une thérapeutique.

En appliquant le procédé de l'invention, un gêne connu comme étant responsable d'une certaine maladie est inséré d'une manière ciblée dans le génome d'une cellule E.S. L'animal transgénique qui est produit à la suite présente un modèle utile de cette maladie.

Si nécessaire, et comme décrit plus haut, les fonctionnements génétiques normaux peuvent être sensiblement maintenus, malgré l'insertion du gène étranger.

Une autre application du procédé de l'invention consiste à insérer un gène d'insertion qui est facilement décélé e.g. le gène lac Z et qui peut donc jouer le rôle d'un marqueur cellulaire. De cette manière, des études de filiation e.g. chez des animaux de concours sont facilitées, et la race peut être suivie.

L'insertion du gène lac Z comme gène d'insertion rend aussi possible des études de promoteur. Grâce à la possibilité de déceler l'activité β-galactosidase, l'activité et la spécificité de différents promoteurs endogènes peuvent être étudiées en ciblant différents sites dans le même type ou différents types de cellules. Les mêmes études pourront être effectuées sur un organisme entier, au cours du développement ou à l'état adulte, en utilisant les techniques d'animaux chimères ou transgéniques.

Les inventeurs ont constaté d'une manière surprenante que la fréquence de recombinaison homologue n'est pas affectée par l'insertion de fragments de taille importante, par exemple le Lac Z. Cette observation a suggéré aux inventeurs que la technique de recombinaison homologue serait bien adaptée à l'insertion d'autres gènes hétérologues qui sont de taille importante.

Grâce à la possibilité de modifier le génome d'un animal, le procédé de l'invention peut également être utilisée en tant que "thérapie génique". Les utilisations les plus évidentes consisteraient à inactiver les gènes de récepteurs d'agents infectieux (virus ou bactéries) ou toxiques. Si cette mutagénèse s'avérait léthale, il faudrait rétablir la fonction perdue sans rétablir la sensibilité aux agents nuisibles. Un gène modifié codant pour un tel récepteur pourrait être réintroduit dans la cellule mutée, à moins que la modification puisse être provoquée par la recombinaison homologue. Cette modification du patrimoine génétique conférerait à l'animal une immunité contre la maladie considérée.

Ce protocole peut aussi intervenir dans le cadre d'auto-greffe. Des cellules, malades ou saines, prélevées sur un patient, pourraient être soignées et immunisées, puis réimplantées chez le même individu.

La technique de l'invention se prête aussi aux études d'activité de produits pharmaceutiques présumés avoir une activité à l'égard des produits d'expression d'un gène pathologique lié à une maladie. Dans ce cas, le gène d'insertion est constitué par le gène pathologique et on administre à l'animal transgénique le produit pharmaceutique en vue d'évaluer son activité sur la maladie.

L'invention va être illustrée en faisant référence au plasmide pGN et son utilisation dans l'insertion ciblée d'un gène étranger (lac Z, codant pour l'enzyme β-glactosidase d'E.Coli) dans le génome d'une cellule d'E.S. de souris. Le gène lac Z a été choisi en raison du fait que son expression peut être facilement décelée et est simplement à titre illustratif.

La phase codante de l'enzyme β-galactosidase d'E.Coli (lac Z; 1-3057), fusionnée avec une séquence génomique (7292-3) du gène murin Hox.3-1 (Ref. 1), débute par le codon d'initiation de ce gène. En effet, la séquence qui précède le codon d'initiation de Hox-3.1 est identique à la séquence consensus observée chez les vertébrés (Ref. 2), permettant ainsi un meilleur taux de traduction de la β-galactosidase dans les cellules de vertébrés. Le gène lac.Z est suivi d'un signal de polyadénylation par exemple du virus SV 40, comme la plupart des gènes eucaryotes, afin de stabiliser les ARN_{S} messagers.

L'activité de la β-galactosidase d'E.Coli, qui est fonctionnelle dans les cellules eucaryotes, peut être décelée de différentes manières. Des cellules exprimant le gène lac Z prennent une coloration bleue, après fixation, en présence de X-Gal, qui est un substrat de la β-galactosidase (Ref. 3). Un nouveau substrat, le FDG (flurorosceine di-β-galactopyranoside), permet de déceler et de doser l'activité β-gal. tout en gardant les cellules vivantes (Ref. 4). Les cellules exprimant lac Z accumulent un produit fluorescent et peuvent être isolées à l'aide d'un trieur de cellules ou FACS (fluorescence-activated cell sorter).

L'unité de transcription du gène de résistance à la néomycine provient, en grande partie, du plasmide pRSV neo (Ref. 5). Le LTR (long terminal repeat) du virus du sarcome de Rous procure des séquences activatrice et promotrice très puissantes dans de nombreuses cellules eucaryotes (Ref. 6). Du transposon bactérien Tn5, viennent un promoteur actif dans E.Coli et la phase codante de l'enzyme phosphotransférase (Ref. 7), qui est suivie du signal de polyadénylation du virus SV40. Le même gène sous le double contrôle des promoteurs RSV et Tn5 peut conférer la résistance à la néomycine ou la kanamycine aux bactéries et la résistance au G418 aux cellules eucaryotes.

Par l'effet d'une simple mutation ponctuelle, l'unité B des séquences activatrices (enhancer) de la souche PyEC F9.1 du virus du Polyome est devenue beaucoup plus active dans différents types de cellules, et en particulier dans les cellules de carcinome embryonnaire (EC) (Ref. 8). Deux copies de cet enhancer Py F9.1 ont été insérées en tandem dans le plasmide pGN, en amont du LTR-RSV, et dans l'orientation "promoteur tardif" de la région régulatrice du Polyome.

Afin d'améliorer le taux de traduction de la phosphotransférase, la séquence précédent le codon d'initiation a été modifiée lors d'une mutagénèse par oligonucleotide. Ainsi la séquence T T C G C A U G est devenue G C A C C A U G, correspondant beaucoup mieux à la séquence consensus d'initiation de la traduction chez les vertébrés (Ref. 2).

Les améliorations apportées à l'unité de transcription du gène de résistance à la néomycine ont pu être estimées en transfectant des cellules souches embryonnaires (ES) de souris. A molarité égale en plasmide, une construction avec les enhancer Py F9.1 a produit 7,5x plus de clones résistants au G418 que le pRSV neo et 2 à 3x plus que le pMC1 Neo décrit par Capecchi et al (réf. 13). A nouveau, le nombre de clones a été augmenté 60x, soit 450x par rapport au pRSV neo, en modifiant la séquence d'initiation de la traduction. La recombinaison homologue peut être un événement assez rare, selon les conditions expérimentales appliquées (p. ex 1/1000 pour HPRT, réf. 13). Un vecteur présentant une efficacité de sélection élevée est donc très utile, d'autant plus que les conditions d'électroporation donnent lieu principalement à l'intégration d'une seule copie.

Le plasmide pGN contient, en outre, une origine de réplication bactérienne de tue colE1, pBR322, qui permet les clonages et les préparations dans E.Coli.

Enfin, un site de clonage multiple (M.C.S.), synthétisé in vitro, qui ne contient que des sites de coupure uniques dans pGN, a été inséré en amont de lac Z, afin de faciliter les utilisations de ce plasmide.

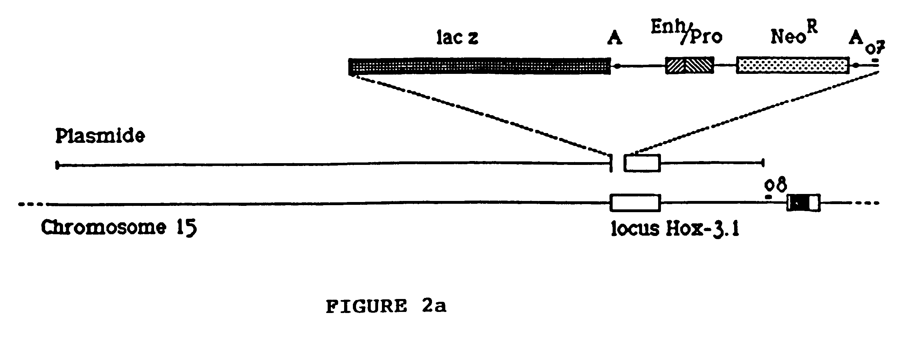

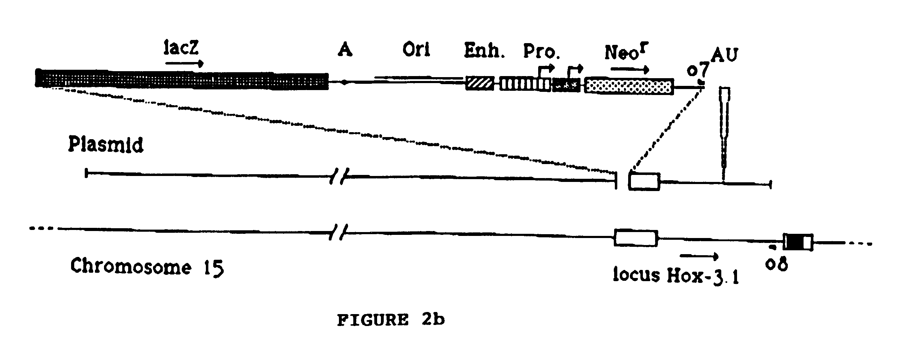

Les séquences "flanquantes" plasmidiques qui provoquent la recombinaison homologue sont ajoutées aux extrémités du plasmide pGN après linéarisation du plasmide en amont de lac Z, par un site du MCS (voir fig. 2). En l'occurence, les séquences flanquantes choisies sont homologues aux séquences chromosomiques issues de Hox-3.1 devant ultérieurement intervenir dans la recombinaison homologue.

La figure 2 situe la molécule construite à partir du plasmide pGN par rapport au gène Hox-3.1. Dans ce cas, une recombinaison entre les séquences plasmidiques et chromosomales de Hox-3.1 résulterait en une insertion au début de la phase codante de ce gène, donc à son inactivation totale.

Le plasmide pGN rassemble plusieurs avantages pour cette méthodologie, qui est applicable à n'importe quel gène. L'évènement de recombinaison homologue pouvant être assez rare (de l'ordre de 1 pour 1000 intégrations non-homologues), il est nécessaire de pouvoir analyser un grand nombre de clones dont la résistance au G418 soit suffisament forte pour s'exprimer dans n'importe quelle partie du génome. Les modifications apportées à l'unité de transcription de la phosphotransférase répondent parfaitement à ces problèmes. La méthode de mutagénèse par recombinaison homologue équivaut à inactiver un gène par une insertion ou une substitution, mais le plasmide pGN présente l'avantage supplémentaire de pouvoir substituer l'expression de la β-galactosidase à celle du gène muté. Enfin, le MCS facilite les clonages de fragments génomiques.

### EXEMPLES :

### I - Construction du plasmide pGN

Les plasmides intermédiaires sont numérotés selon leur étape.

### 1° étape :

Insertion d'un site Xho I dans le site Bgl I de pRSV neo
Insertion d'un linker Xho I dans le site Bgl I de pRSV neo, rempli par le fragment Klenow de l'ADN polymérase d'E.Coli.

### 2° étape :

Insertion d'un site Cla I dans le site Nde I du plasmide p1
Insertion d'un linker Cla I dans le site Nde I de p1, rempli par la polymérase Klenow.

### 3° étape :

Insertion d'enhancer Py F9.1 dans le site Cla I du plasmide p2
Insertion d'enhancer Py F9.1 Pvu II-Pvu II isolé par un site unique, Acc I, dans le site Cla I de p2. Sélection d'un clone contenant deux enhancers orienté dans le sens "promoteur tardif".

### 4° étape :

Déletion Sma I-Hpa I du plasmide p3
Les deux enzymes, donnant des extrémités "bouts-francs", peuvent être ligués directement. Cette délétion enléve l'intron de l'antigène t de SV 40, qui n'est pas très utile, et diminue de manière appréciable la taille de l'unité de transcription de la phosphotransférase.

### 5° étape :

Insertion d'un site Xho I dans le site Bam HI de pCH110
Insertion d'un linker Xho I dans le site Bam HI du plasmide pCH 110 (Pharmacia), rempli par la polymlérase Klenow.

### 6° étape :

Insertion du 3' lac.Z-polyA SV 40 dans le plasmide p4
La partie 3' de la phase codante de la β-galactosidase, suivie du signal de polyadénylation du virus SV 40 est isolée du plasmide p5 par les sites Xho I-Aat II et clonée dans le plasmide p4 par les mêmes sites.

### 7° étape :

Insertion du 5' lac.Z dans le vecteur KS-
La partie 5' de la phase codante de la β-galactosidase est isolée du plasmide pMC 1871 (Pharmacia) par les sites Pst I-Sac I et clonée dans le vecteur KS- (Stratagene) par les mêmes sites.

### 8° étape :

Fusion d'une séquence génomique Hox-3.1 avec le 5' lac.Z
Une séquence génomique du gène Hox-3.1, clonée dans le vecteur KS-, est purifiée par digestions successives par l'enzyme Sac I, puis par la nucléase Mung bean et enfin par l'enzyme Apa I. Cet insert est fusionné avec la partie 5' de la phase codante de la β-galactosidase par clonage dans le plasmide p7 digéré par Apa I-Sma I. La protéine ainsi fusionnée contient le codon d'initiation de la traduction du gène Hox-3.1 suivi de la phase codante de la β-galactosidase (vérifiée ensuite par séquençage).

Met Ser Ser Ile Pro Gly Asp Pro
CCAGC ATG AGC TCC ATT CCC GGG GAT CCC
GGTCG TAC TCG AGG TAA GGG CCC CTA GGG
⇓ Sac I
CCAGC ATG AGC T ⇓ Sma I
GGTCG TAC
⇓ nucléase Mung bean
CCAGC ATG GGG GAT CCC
GGTCG TAC ⇔ CCC CTG GGG
Met Gly Asp Pro
CCAGC ATG GGG GAT CCC
GGTCG TAC CCC CTA GGG

### 9° étape :

Insertion de Hox-3.1-5' lac.Z dans le plasmide p6
La fusion Hox-3.1-5' lac.Z est isolée du plasmide p8 par les sites Apa I-Sac I et clonée dans le plasmide p6 par les mêmes sites. Ce clonage a pour effet de reconstituer la phase codante de la β-galactosidase dans sa totalité.

### 10° étape :

Insertion du gène Neo^{R} dans le vecteur KS+
Le gène de résistance à la néomycine (promoteur bactérien et phase codante de la phosphotransférase) est isolée du pRSV neo par les sites Hind III-Eco RI et clonée dans le vecteur KS+ (Stratagene).

### 11° étape :

Mutagénèse de la séquence d'initiation de Neo^{R} dans p10
La séquence d'initiation de la traduction de la phnosphotransférase est modifiée pour être identique à la séquence consensus observée chez les vertébrés et permettre ainsi un taux supérieur d'initiation de la traduction donc une résistance accrue au G418 pour les cellules de mammifères. La modification crée également un site Apa LI qui permet de contrôler l'efficacité de la mutagénèse. 
Apa LI
GTTTCGCATG ⇒ GTGCACCATG
Un oligonucléotide (CTTGTTCAATCATGGTGCACGATCCTCA) comportant une région de misappariement avec la séquence du pRSV neo (soulignée) est synthétisé (Gene Assembler, Pharmacia) puis phosphorylée par la polynucléotide kinase du bactériophage T4. Une matrice simple brin du plasmide p10 est préparée grâce à l'origine f1 du plasmide KS+ et hybridée avec l'oligonucléotide de mutagénèse. Le deuxième brin est synthétisé et réparé par la polymérase Klenow et l'ADN ligase du bactériophage T4. Aprés transformation de bactéries, les clones mutés sont criblés à l'aide de l'oligonucléotide marqué au ³²P. La mutagénèse a été vérifiée en digérant par Apa LI ainsi que par séquençage.

### 12° étape :

Remplacement de la séquence d'initiation dans le plasmide p9
Un fragment contenant la séquence modifiée d'initiation de la traduction du gène de résistance à la néomycine est isolée du plasmide p11 par les enzymes Hind III-Eag I et clonée dans le plasmide p9 par les mêmes sites.

### 13° étape :

Insertion du site de clonage multiple dans le plasmide p12
Deux oligonucléotides complémentaires sont synthétisés (Gene Assembler, Pharmacia) puis phosphorylés. Après appariement, le MCS est cloné dans les sites Apa I- Sac II du plasmide p12 grâce à ses extrémités cohésives 
Xma I     Asp 718
Xmn I
Apa I     Sma I     Kpn I     Xba I     Nsi I     Sac II
5' CCCCGGGGGTACCTCTAGAATGCATTCCGC 3'
3' CCGGGGGGCCCCCATGGAGATCTTACGTAAGG 5'
Le site de clonage multiple a également été vérifié par séquençage.

### II - Addition des séquences "flanquantes" aux extrémités du plasmide pGN linéarisé en amont de lac.Z' par un site du M.C.S

Les séquences flanquantes utilisées ont été choisies en fonction du site d'insertion souhaité (par exemple, Hox-3.1, voir Fig. 2 a et b pGMA et pGMD).

Dans la construction du plasmide de mutagénèse pGMD, deux bras d'ADN homologue au locus Hox-3.1 ont été clonés aux sites Apa I-Nsi I et Nsi I-Sac II du vecteur pGN. Le bras 5' commence au site Sac II (CCGCGG)au nucléotide 219 de l'ADNc c21 de Hox-3.1. Ce fragment s'étend sur 6.8 kb en 5' jusqu'au premier site BamHI. Le bras 3' commence au site Apa l (GGGCCC) au nucléotide 885 de l'ADNc c21. Ce fragment s'étend sur 1.5 kb en 3' jusqu'au premier site PstI. Un linker NsiI a été inséré dans le site BamHI du fragment 5' et dans le site PstI du fragment 3'. Les bras 5' et 3' ont été clonés dans le vecteur pGN dans les sites Nsi I-Sac II et Apa I-Nsi I, respectivement. La séquence de l'ADNc de Hox-3.1 c21 est publiée (réf. 1).

Le plasmide de mutagénèse est linéarisé par digestion avec Nsi I avant électroporation de cellules E.S. Ses extrémités sont formées des deux bras génomiques clonés aux sites Apa I-Nsi I et Nsi I-Sac II du vecteur pGN.

Le plasmide pGMD ne présente pas de signal de polyadénylation après le gène de résistance mais, en revanche, présente une séquence riche en AU responsable d'une dégradation sélective de mRNA, insérée dans la séquence de l'intron du Hox-3.1 du plasmide.

Un autre plasmide de mutagénèse, pGMA, présente la même structure que le pGMD mais contient les signaux de polyadénylation et de terminaison de transcription du SV40 et ne présente pas la séquence AU de dégradation de mRNA en aval du gène Neo^{r}. Ces modifications avaient pour but de réduire le taux de transcrits de Neo^{r} dans des clones issus d'intégration au hasard. En revanche, des clones issus d'événements de recombinaison homologue entre pGMD et un locus Hox-3.1 devrait avoir une croissance inaltérée pendant la sélection au G418, la séquence AT de dégradation de mRNA étant éliminée par le procédé de recombinaison même, ou epissée avec l'intron Hox-3.1.

Dans les étapes expérimentales qui suivent, le protocole décrit par Thompson et al. 1989, a été suivi pour la production d'animaux chimères.

### III - Transfection de cellules embryonnaires de souris

La méthode décrite par Thompson et al. 1989, a été utilisée pour transfecter des cellules embryonnaires de souris. L'utilisation de la technique de l'électroporation assure l'introduction d'une seule copie du gène étranger (lac.Z) par cellule. Après transfection, plusieurs clones exprimant la β-galactosidase ont été isolés.

Les plasmides de mutagénèse pGMD et pGMA ont été linéarisés et introduits par électroporation dans des cellules E.S. afin de favoriser l'insertion d'une copie seulement dans le génome (réf. 11).

Les transfections initiales ont été effectuées pour comparer l'efficacité de ciblage du Hox-3.1 des plasmides pGMA et pGMD (voir tableau I).

**Tableau I**

| Recombinaison homologue dans le gène Hox-3.1 | | | | |
|---|---|---|---|---|
| Exp. | Plasmide de mutagénèse | N° de l'ensemble analysé | Nb de clones formant l'ensemble | Nb de résultats P.C.R. positifs |
| I | pGMA | 3 | 600 | 0(2) |
| II | pGMD | 5 | 250 | 3(5) |
| III | pGMD | 84 | 2-3 | 5(5) |

La lignée cellulaire E.S. "C.C.E." (réf. 16) a été maintenue d'une manière continue sur des couches nourricières fibroblastiques (réf. 17). Pour les expériences I et II, 1.5 x 10⁷ cellules E.S. dans 1.5 ml HeBS ont été électroporées (réf. 11) à 200 V, avec 40 mg de plasmide linéarisé, puis étalées sur quatre boîtes de cultures (diamètre 100 mm). Pour l'expérience III, le choc a été effectué dans les mêmes conditions mais un quart des cellules ont été étalées sur quatre plaques à 24 puits. Le lendemain, 250 »g ml⁻¹ G418 ont été ajoutés. Chaque transfection a donné lieu à environ 2400 clones avec pGMA et environ 1000 clones avec pGMD.

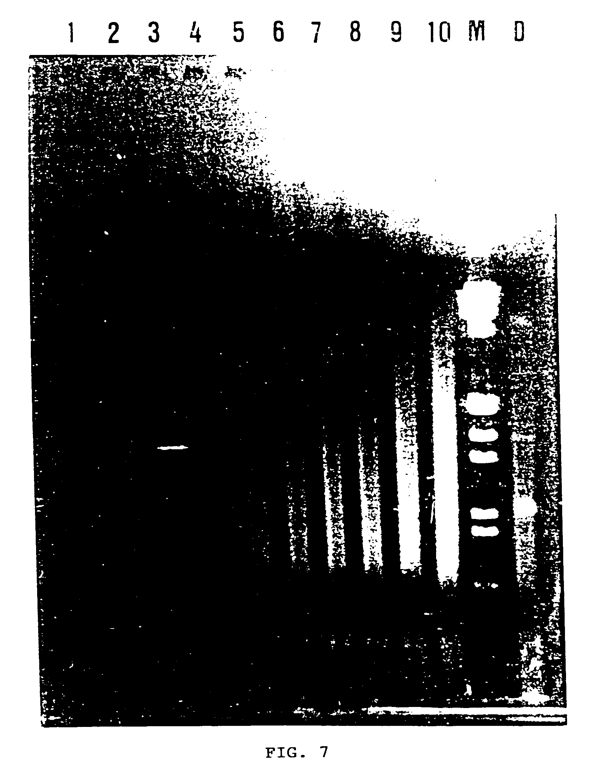

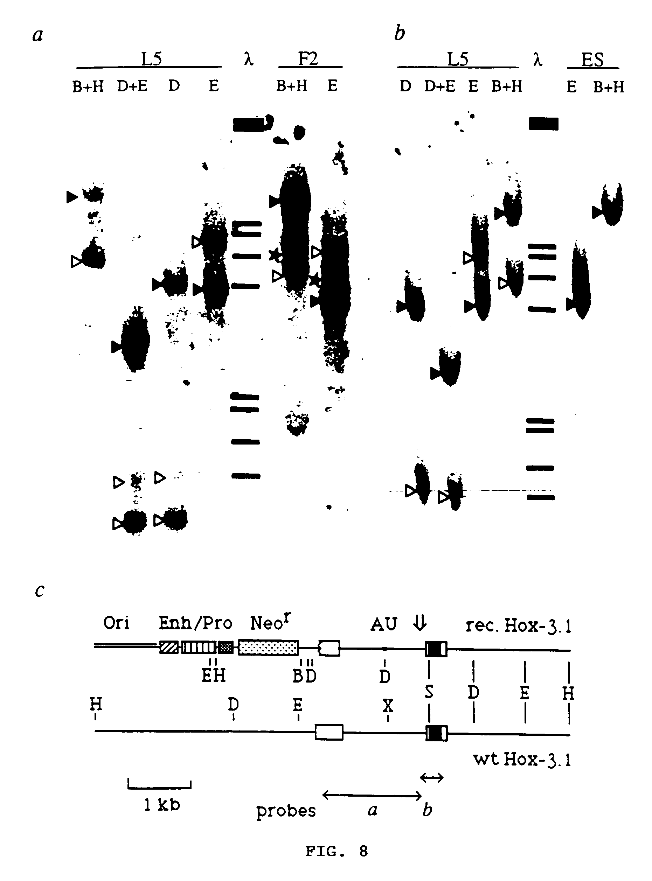

Le nombre moyen de clones de cellules E.S. résistantes au G418 dans chaque ensemble est indiqué dans le tableau I, ainsi que le nombre d'ensembles donnant un résultat positif avec la technique P.C.R. Un résultat positif signifie q'une bande de 1.6 Kb a pu être observée sur un gel d'agarose coloré de bromure d'ethidium (voir fig. 7). Le nombre d'ensembles donnant un signal positif après une analyse de Southern du mélange P.C.R. et hybridation d'une sonde spécifique qui ne contenait pas les séquences des amorces est indiqué entre parenthèses (fig. 8).

### Détection de la recombinaison homoloque avec la P.C.R.

P.C.R. a été effectuée sur 10⁵ cellules d'un ensemble de 250 clones de la transfection II (voir voie D de la fig. 7). Dans les autres voies, quatre ensembles de la transfection III ont été analysés ensemble en mélangeant environ 4 x 5000 cellules. Les amorces 07 et 08 utilisées dans la P.C.R. entourent la séquence 3' Hox-3.1 du plasmide de mutagénèse (fig. 2). Le fragment de 1.6 Kb recouvrant cette séquence 3' ne peut être amplifié que dans le cas d'une recombinaison homologue. Les voies 2, 3 et D illustrent des résultats positifs.

L'ADN de clones E.S. a été préparé au moment de la réplique sur filtre en utilisant la méthode "boiling-proteinase K digestion boiling" (réf. 18). 40 cycles d'amplification (40 secondes à 94°C, 1 minute à 60°C, 7 minutes à 72°C) ont été effectués dans un mélange réactionnel de 100 »l, contenant 67 mM Tris-HCL (pH 8.6), 16.7 mM (NH₄)₂SO₄, 6.7 mM MgCl₂, 10 mM 2-mercaptoethanol, 0.01 % (p/v) gélatine, 200 »M dATP, dTTP et dCTP, 100 »M dGTP, 100 »M 7-deaza dGT, 600 ng de chaque amorce (07 : AACTTCCCTCTCTGCTATTC et 08 : CAGCAGAAACATACAAGCTG) et 3U polymérase Taq (Perkin Elmer Cetus), couvert de 100 »l paraffin. La moitié du mélange de réaction a été appliquée sur un gel d'agarose 0.7 % coloré au bromure d'ethidium. Le marqueur de taille est un digest Eco RI + Hind III d'ADN lambda.

### Analyses de Southern

Trois clones indépendants de cellules E.S. contenant le Hox-3.1 muté (identifié par P.C.R.) ont été isolés des ensembles positifs en utilisant des pipettes. Leur ADN a été examiné par analyse de Southern après digestion avec les enzymes de restriction indiqués dans la figure 8, afin de confirmer le ciblage spécifique et de faire la distinction entre les loci recombinés et sauvages. Deux sondes différentes ont été utilisées dans l'analyse de l'extrémité 3' des loci Hox-3.1 dans les clones mutés et dans les cellules E.S. non-mutées agissant comme témoin (fig. 8 c). La première sonde "a" était contenue dans les séquences Hox-3.1 du plasmide de mutagénèse et démontrait le nombre d'intégrations de vecteur et leurs liaisons physiques. Un des trois clones recombinés contenait en outre une copie du plasmide intégrée au hasard (fig. 8 a, clone F2). La deuxième sonde "b" qui n'était pas contenue dans le vecteur de mutagénèse faisait la distinction entre les allèles Hox-3.1 recombinées et sauvages (fig. 8 b). Le locus recombiné Hox-3.1 présentait, avec les deux sondes, l'image d'hybridation attendue à partir des cartes de restriction du vecteur de mutagénèse et du locus intact. En outre, l'existence de deux domaines de recombinaison dans le bras 3' du vecteur a été confirmée par la présence ou l'absence de la séquence AT dans le locus Hox-3.1 recombiné (par exemple fig. 8, clone L5). L'extrémité 5' du locus Hox-3.1 a également été analysée pour l'événement de recombinaison homologue. Des enzymes de restriction ne présentant pas de sites dans la séquence Hox-3.1 5' de 6.8 Kb du vecteur de mutagénèse ont été utlisés dans la digestion des ADNs des clones recombinés. Ces ADNs ont ensuite été soumis à une électrophorèse dans un champ pulsé pour différencier les fragments de poids moléculaire élevé. Une analyse de Southern de ce gel a également indiqué les allèles recombinées correctement et les allèles Hox-3.1 sauvages, en utilisant une sonde présentant une séquence en amont du plasmide de mutagénèse.

Les analyses de Southern ont démontré q'une allèle du gène Hox-3.1 a été recombinée comme prévu. La recombinaison homologue était équivalente à un double "crossing-over" entre les bras génomiques du plasmide de mutagénèse et les séquences chromosomales homologues (Fig. 2).

Dans les clones recombinants, le gène lac Z a été placé sous le contrôle des séquences promotrices et régulatrices du Hox-3.1 en amont du codon AUG, mais les signaux 3' de maturation de mRNA provenaient du SV40. Dans ces clones recombinés, l'expression de lac Z n'était pas décelable par coloration avec β-Gal, ce qui est cohérent avec l'absence de transcription de Hox-3.1 dans des cellules E.S. déterminée par analyse de protection de RNase. L'activité de β-Gal pouvait être induite dans certaines cellules après 3 ou 4 jours de culture en présence de 5.10⁻⁷M acide rétinoïque, conditions connues comme induisant la transcription de Hox-3.1 (réf. 19).

En utilisant le vecteur de mutagénèse pGMA, qui présente une homologie totale de 8.3 Kb ADN avec le locus Hox-3.1, un fragment de 120 pb a été remplacé par une insertion de 7.2 Kb. La fréquence de ce remplacement ciblé (1/900) est comparable à celle obtenue récemment (1/1000) avec HPRT (réf. 13) ou avec En-2 (1/260) (réf. 20), le fragment hétérologue inséré étant cependant dans ces derniers cas d'une taille beaucoup moins importante (1.1 et 1.5 Kb respectivement). D'une manière surprenante, il a été constaté qu'une fréquence de recombinaison homologue très élevée (1/40) a pu être obtenue avec le vecteur pGMD. L'élimination des signaux 3' de maturation de mRNA et l'addition de la séquence de dégradation de mRNA au gène de résistance à la néomycine a eu pour effet de réduire le nombre total de clones résistants au G418 par 2.4 (tableau I). Le rapport de ciblage spécifique était presque 10 fois plus élevé (900/40). Le mécanisme de recombinaison homologue même a dû être affecté dans les expériences avec pGMD. Une explication possible de ces résultats serait qu'une séquence AT de 51 pb pourrait fournir, in vivo, une boucle ouverte dans le plasmide de mutagénèse à cause de sa température de fusion plus basse. Si les séquences Hox-3.1 voisines du pGMD peuvent être influencées par cette ouverture, de chaque côté de la région AT, elles pourraient réagir d'une manière plus efficace, à l'état simple-brin, avec le locus chromosomal Hox-3.1. Le modèle de recombinaison mitotique chez la levure suggère qu'il serait initié par un tel échange de brins, bien que le mécanisme de recombinaison homologue reste inconnu chez les eukaryotes plus complexes.

La figure 8 montre les résultats de l'analyse de Southern effectuée sur des clones individus positifs (L5 et F2) et des cellules E.S. (C.C.E.).

Les sondes utilisées n'hybrident qu'avec des séquences Hox-3.1 incluses dans le vecteur (a) ou exclues du vecteur de mutagénèse (b). L'image d'hybridation du locus Hox-3.1 recombiné (triangles ouverts) se distingue clairement du locus sauvage (triangles noirs). Les étoiles indiquent les bandes d'hybridation d'une copie du plasmide qui s'est intégrée au hasard. Le marqueur de taille est un digest Eco RI + Hind III d'ADN lambda.

La figure 8(c) montre les cartes de restriction des allèles Hox-3.1 recombinées (rec.) et sauvages (wt). Les parties du vecteur de mutagénèse et du locus Hox-3.1 sont indiquées avec les mêmes symboles que ceux utilisés dans la figure 2. Dans ce cas, la séquence AT a été intégrée par recombinaison homologue. La flèche verticale indique l'extrémité 3' du plasmide de mutagénèse. La localisation des sondes "a" et "b" utilisées dans l'analyse de Southern est également indiquée. Les abréviations utilisées dans la figure 8 sont les suivantes : B, Bam HI ; D, Dra I, E, Eco RI ; H, Hind III ; S, Sal I ; X, Xho I.

### IV - Production d'embryons chiméres

Une microinjection dans des blastocystes a été effectuée avec deux clones E.S. recombinants contenant un allèle Hox-3.1 intact et un allèle recombiné, ces clones ne contenaient aucune autre copie du plasmide de mutagénèse. Les kariotypes de ces cellules étaient normaux.

Dix à quinze cellules mutées ont été microinjectées par blastocyste. Après réimplantation dans des mères porteuses, les embryons ont été recueillis à 9.5, 10.5 et 12.5 jours p.c. et analysés pour expression de lac Z. La grille de transcription de Hox-3.1 à ces stades avait été déterminée au préalable par analyse d'hybridation in situ (réf. 1). Les transcrits Hox-3.1 sont détectables pour la première fois au stade de gastrulation tardive et sont répartis dans tous les tissus de la partie postérieure de l'animal. Plus tard, la répartition devient progressivement limitée dans l'espace et spécifique au tissu. Au stade de 12.5 jours p.c., la transcription est localisée dans la région cervicale du tube neural, au niveau du coeur. Au cours de l'embryogenèse, la répartition de la transcription de Hox-3.1 subit donc des modifications. Le stade 10.5 jours p.c. semble être une période de transition, la transcription ayant lieu à la fois dans les deux régions arrières et dans le tube neural cervical.

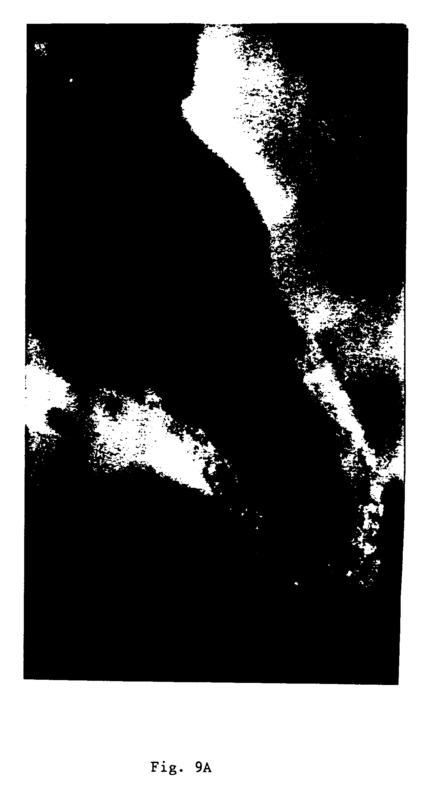

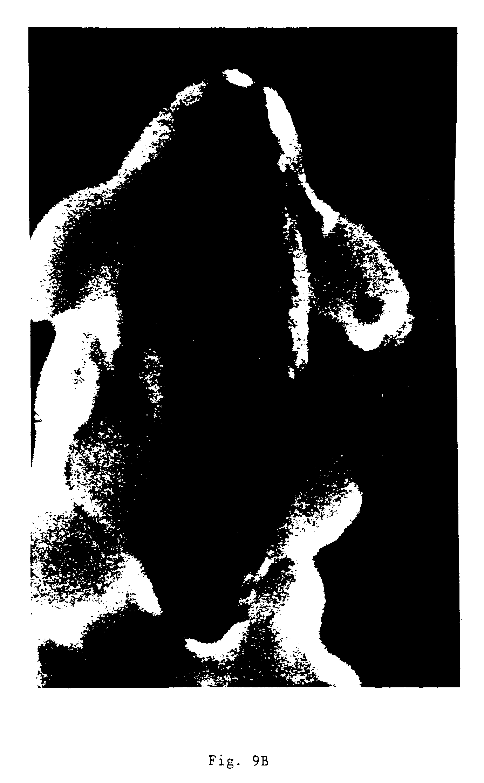

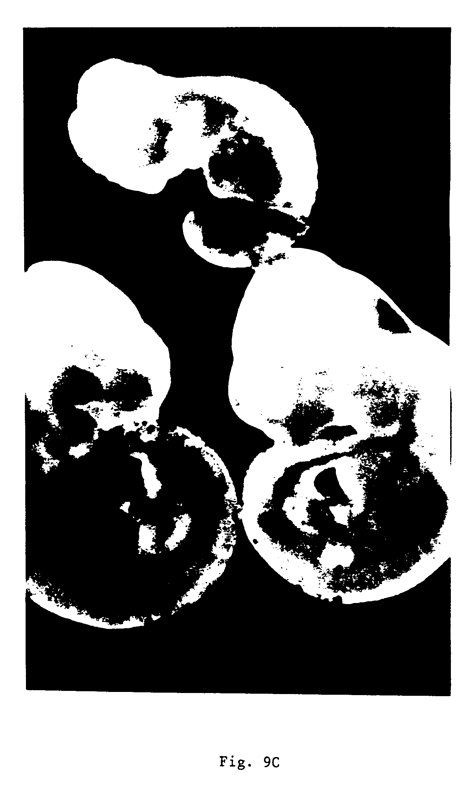

Dans des embryons chimériques de 9.5 et 10.5 jours p.c., la partie caudale au bourgeon postérieur présentait une activité β-Gal intense, tandis que le marqueur n'a jamais été détecté dans la région thoracique antérieure ou la tête (Fig. 9a). Dans la région postérieure, des cellules colorées par le β-Gal ont été observées dans tous les tissus et de toutes les couches embryonnaires. Entre les deux bourgeons qui donnent les membres, des cellules colorées étaient réparties dans des zones restreintes, dans l'ectoderme superficiel (Fig. 9b), comme dans les régions arrières (Fig. 9c) et, en forme de lignes étroites ou rayures, dans le tube neural (Fig. 9b). Ces rayures présentaient une répartition irrégulière et asymétrique sur la paroi du tube neural. La transcription de Hox-3.1 n'a pas été détectée dans la couche mince de cellules vers la fermeture du tube neural. Ces cellules n'ont peut-être pas résisté aux traitements appliqués lors de l'hybridation in situ. Il a été observé que les cellules de l'ectoderme neural font partie, très tôt, de parties différentes du système nerveux et migrent dans une direction radiale, suivant des mouvements latéraux étroits (réf. 21). Ces résultats sont donc cohérents avec cette observation.

L'expression de Lac Z a donc illustré correctement la première partie de la transcription de l'homéogène Hox-3.1, c'est-à-dire dans tous les tissus des régions caudales des embryons de 9.5 et 10.5 jours p.c., et a fourni de nouvelles informations concernant le mode de transcription de Hox-3.1.

En revanche, l'expression de Lac Z n'a pas été observée dans les régions cervicales du tube neural d'embryons chimères de 12.5 jours, ni dans la région antérieure d'embryons de 10.5 jours ; ceci n'était pas le résultat attendu à partir des études d'hybridation in situ. La phase ultérieure de transcription de Hox-3.1 observée à partir du jour 10.5 dans les zones très localisées du tube neural n'était pas mise en évidence par l'activité de β-Gal. Une explication possible pour ce résultat serait que, bien que l'expression de Lac Z soit sous le contrôle du promoteur Hox-3.1, les séquences 3' du Hox-3.1 sont absentes dans le gène reporteur. Il est possible que des séquences 3' du codon d'initiation AUG du Hox-3.1 aient une influence sur l'expression tardive de Hox-3.1 dans le domaine antérieur. Un effet de "dosage de gène" pourrait aussi expliquer ce résultat. L'autoactivation de plusieurs homéogènes chez Drosophila a été démontrée génétiquement ou suggérée par la formation de complexes entre l'ADN et les protéines des homeobox.

Si le composant tardif de la transcription de Hox-3.1 dans le tube neural est maintenu par un mécanisme semblable, l'inactivation d'un allèle pourrait avoir un effet dominant dans les celules de l'ectoderme neural. Puisqu'un allèle seulement produirait la protéine Hox-3.1, le signal d'activation serait dilué sur les deux promoteurs. La réduction d'autoinactivation dans les deux loci pourrait ainsi conduire à un arrêt total de l'initiation de transcription. Ceci pourrait expliquer pourquoi aucune expression de Lac Z n'a été détectée dans la région cervicale du tube neural d'embryons de 10.5 et 12.5 jours.

### V - Passage de la modification dans la lignée cellulaire germinale : production d'animaux transgéniques

Les effets en F₁ et en F₂ de la modification apportée par l'insertion ciblée ont été observés après reproduction des chimères. Le passage de la modification dans la lignée cellulaire germinale a été constaté.

### BIBLIOGRAPHIE

1. Le Mouellic, H., Condamine, H. et Brûlet, P. (1988). Genes & Development. 2, 125-135.
2. Cavenir, D.R. (1987). Nucleic Acids Res. 15, 1353-1361.
3. Sanes, J.R. Rubenstein, J.L.R. et Nicolas, J.F. (1986) EMBO J. 5, 3133-3142.
4. Nolan, G.P., Fiering, S., Nicolas, J.F. et Herzenberg, L.A. (1988) Proc. Natl. Acad. Sci. USA 85, 2603-2607.
5. Gorman, C., Padmanabhan, R. et Howard, B.H. (1983). Science. 221, 661-553.
6. Gormann, C.M., Merlino, G.T. Willingham, M.C. Pastan, I. et Howard, B. H. (1982) Proc. Natl. Acad. Sci. USA 79, 6777-6781.
7. Southern, P.J. et Berg, P. (1982) J. Mol. Appl. Genet 1, 327- 341.
8. Herbomel, P., Bourachot, B. et Yaniv, M. (1984). Cell. 39, 653-662.
9. Robertson, E.J. (1987). Teratocarcinomas and embruonic stem cells: A practical approach. IRL Press, Oxford.
10.Kahn, A. (1988). Médecine/Sciences 4, 515-518.
11.G. Chu, Hayakawa H., Berg. P. (1987) Nucleic Acid Research 15, Nr. 3, 1311-1326.
12.Thompson, S., Clarke, A.R., Pow, A.M., Hooper, M.L., Melton, D.W., (1989) Cell, 56, 313-321.
13.Thomas, K.R.,Capecchi, M.R., (1987) Cell, 51 503-512.
14. Evans, M.J., Kaufmann, M.H. (1981) Nature, 292, 154-155,
15.Robertson, E.J., (1986) Trends in Genetics, 9-13
16.Robertson, E., Bradley, A., Kuehn, M. & Evans, M. Nature 323, 445-448 (1986).
17.Robertson, E.J. in Teratocarcinomas and Embryonic Stem Cells (ed. Robertson, E.J.) 71-112 (IRL, Oxford, 1987).
18.Kim, H.S. & Smithies, O. Nucleic Acids Res. 16, 8887-8903 (1988).
19.Breier, G., Bucan, M., Francke, U., Colberg-Poley, A.M. & Gruss, P. EMBO J.5, 2209-2215 (1986).
20.Joyner, A.L., Skarnes, W.C. & Rossant, J. Nature 338, 153-156 (1989).
21.Mckay, R. D. G. Cell 58, 815-821 (1989).

## Revendications

1. Procédé de remplacement ou d'insertion spécifique, d'un gène notamment par ciblage d'un ADN, dit ADN d'insertion constitué par la totalité d'un gène, ou par une partie d'un gène susceptible d'être rendu fonctionnel, ou dont le fonctionnement peut être rendu plus efficace, lorsqu'il est recombiné avec un ADN de complément pour alors fournir un gène recombinant complet dans le génome d'une cellule eucaryote, caractérisé en ce que
- le site d'insertion se trouve dans un gène choisi, dit gène receveur choisi, et contenant l'ADN de complément, et en ce que
- l'on transfecte des cellules eucaryotes avec un vecteur contenant un insérat comprenant lui-même l'ADN d'insertion et deux séquences dites "flanquantes" de part et d'autre de l'ADN d'insertion, respectivement homologues à deux séquences génomiques qui jouxtent le site d'insertion souhaité dans le gène receveur,
- l'ADN d'insertion étant hétérologue vis-à-vis du gène receveur, et
- les séquences flanquantes, étant choisies parmi celles qui constituent le susdit ADN de complément et qui autorisent, par recombinaison homologue avec des séquences correspondantes du gène receveur, la reconstitution d'un gène recombinant complet dans le génome de la cellule eucaryote, à condition que lorsque l'ADN d'insertion est constitué d'une séquence codante, il ne s'agit pas de la séquence codante d'un gène codant pour un agent de sélection.

2. Procédé de remplacement ou d'insertion spécifique, d'un gène notamment par ciblage d'un ADN, dit ADN d'insertion constitué par la totalité d'un gène, ou par une partie d'un gène susceptible d'être rendu fonctionnel, ou dont le fonctionnement peut être rendu plus efficace, lorsqu'il est recombiné avec un ADN de complément pour alors fournir un gène recombinant complet dans le génome d'une cellule eucaryote, caractérisé en ce que
- le site d'insertion se trouve dans un gène choisi, dit gène receveur choisi, et contenant l'ADN de complément, et en ce que
- l'on transfecte des cellules eucaryotes avec un vecteur contenant un insérat comprenant lui-même l'ADN d'insertion et deux séquences dites "flanquantes" de part et d'autre de l'ADN d'insertion, respectivement homologues à deux séquences génomiques qui jouxtent le site d'insertion souhaité dans le gène receveur,
- l'ADN d'insertion étant hétérologue vis-à-vis du gène receveur, et
- les séquences flanquantes, étant choisies parmi celles qui constituent le susdit ADN de complément et qui autorisent, par recombinaison homologue avec des séquences correspondantes du gène receveur, la reconstitution d'un gène recombinant complet dans le génome de la cellule eucaryote,
- le gène receveur étant un gène qui, normalement, n'est pas transcrit dans la cellule eucaryote.

3. Procédé selon la revendication 1 ou 2, caractérisé en ce que ledit ADN d'insertion contient soit une séquence codante soit une séquence régulatrice,
- les séquences flanquantes étant choisies afin de permettre par recombinaison homologue selon le cas, soit l'expression de la séquence codante de l'ADN d'insertion entier sous le contrôle des séquences régulatrices du gène receveur, soit l'expression d'une séquence codante du gène receveur sous le contrôle de séquences régulatrices de l'ADN d'insertion.

4. Procédé selon l'une quelconque des revendications 1, 2 ou 3, caractérisé en ce que l'ADN d'insertion contient une séquence codante dépourvue d'élément de régulation, notamment d'un promoteur qui lui est propre.

5. Procédé selon la revendication 3 caractérisé en ce que l'ADN d'insertion comporte une séquence régulatrice.

6. Procédé selon la revendication 5 caractérisé en ce que le promoteur endogène du gène receveur est inactif dans les cellules eucaryotes transfectées.

7. Procédé de remplacement spécifique d'une séquence régulatrice d'un gène, dit gène receveur par ciblage d'un ADN, dit ADN d'insertion, ledit ADN d'insertion contenant une séquence régulatrice, caractérisé en ce que
- l'on transfecte des cellules eucaryotes avec un vecteur contenant un insert comprenant lui-même l'ADN d'insertion et deux séquences dites "flanquantes" de part et d'autre de l'ADN d'insertion, respectivement homologues à deux séquences génomiques qui jouxtent le site d'insertion souhaité dans le gène receveur,
- l'ADN d'insertion étant hétérologue vis-à-vis du gène receveur et,
- les séquences flanquantes étant choisies afin de permettre par recombinaison homologue l'expression d'une séquence codante du gène receveur sous le contrôle de séquences régulatrices de l'ADN d'insertion.

8. Procédé selon la revendication 7 caractérisé en ce que le promoteur endogène du gène receveur est normalement inactif dans les cellules eucaryotes transfectées.

9. Procédé selon l'une quelconque des revendications précédentes, caractérisé en ce que le gène receveur est présent dans le génome de la cellule eucaryote en au moins deux exemplaires.

10. Procédé selon l'une quelconque des revendications précédentes, caractérisé en ce que chacune des séquences flanquantes a une longueur supérieure à 150 paires de bases, et inférieure à la longueur du gène receveur.

11. Procédé selon l'une quelconque des revendications 1 à 10, caractérisé en ce que les cellules eucaryotes sont des cellules souches embryonnaires (E.S.).

12. Procédé selon l'une des revendications précédentes, caractérisé en ce que l'ADN d'insertion est un gène hétérologue ou une partie d'un gène hétérologue à l'espèce transfectée.

13. Procédé selon l'une des revendications précédentes, caractérisé en ce que le vecteur contient des séquences intercalées entre le gène d'insertion et les séquences flanquantes.

14. Procédé selon la revendication 13, caractérisé en ce que les séquences intercalantes contiennent une séquence codant pour un agent sélectif permettant la sélection des transformants et éventuellement un gène marqueur par exemple le LacZ.

15. Procédé selon l'une des revendications précédentes, caractérisé en ce que la transfection est effectuée par électroporation.

16. Procédé selon l'une des revendications précédentes, caractérisé en ce que la technique de Polymerase Chain Reaction (P.C.R.) est utilisée pour amplifier la séquence d'ADN du locus où se fait l'insertion pour vérifier que l'insertion a eu lieu dans le site souhaité.

17. Procédé selon l'une quelconque des revendications précédentes caractérisé en ce que l'ADN d'insertion comporte une partie du gène de l'interféron, de l'interleukine, de gènes de récepteurs tels que le récepteur de l'acide rétinoique β-3-adrénergique ou le récepteur H.I.V, ou d'un gène lié à une maladie, ou du gène lacZ.

18. Procédé selon l'une quelconque des revendications précédentes, caractérisé en ce que l'ADN d'insertion comporte, entre les séquences flanquantes, d'une part une séquence d'ADN destinée à être recombinée avec l'ADN de complément dans le gène receveur pour fournir un gène recombinant, et, d'autre part, une séquence codant pour un agent sélectif permettant la sélection des transformants et un promoteur autorisant l'expression de l'agent sélectif.

19. Procédé de production d'animaux transgéniques, caractérisé en ce que des cellules E.S. sont transfectées par le procédé selon l'une des revendications 1 à 18 et criblées pour l'évènement de recombinaison homologue, à savoir l'intégration correcte du gène étranger, les cellules sont injectées dans des embryons à un stade où ils sont aptes à intégrer les cellules transfectées, par exemple au stade blastocyste, ceux-ci sont ensuite réimplantés dans une mère porteuse et, les individus chimères obtenus au terme de la gestation et chez lesquels est constaté la colonisation par les cellules E.S. de la lignée germinale, sont accouplés pour obtenir des animaux transgéniques hétérozygotes pour le gène remplacé.

20. Séquence d'acide nucléique, dite séquence d'insertion, comprenant :
i) une séquence comprenant tout ou partie d'un gène susceptible d'être rendu fonctionnel ou dont le fonctionnement peut être rendu plus efficace lorsqu'il est recombiné avec un ADN de complément pour alors fournir un gène recombinant complet dans le génome d'une cellule eucaryote, à condition que lorsque la partie du gène est constituée par une séquence codante, il s'agit d'une séquence codante autre que celle d'un gène codant pour un agent de sélection ,
ii) de part et d'autre de la séquence i) deux séquences dites "flanquantes" respectivement homologues aux deux séquences génomiques qui jouxtent le site d'insertion souhaité dans le gène receveur.

21. Séquence d'acide nucléique selon la revendication 20, caractérisée en ce qu'elle comprend, en outre, entre les séquences flanquantes une séquence codant pour un agent sélectif permettant la sélection des transformants et un promoteur autorisant l'expression de l'agent sélectif.

22. Séquence d'acide nucléique selon la revendication 20 ou 21 caractérisée en ce qu'elle comprend, en outre, une séquence éliminée par le procédé de recombinaison homologue, et permettant de sélectionner les transformants issus d'évènements de recombinaison homologue.

23. Vecteur comportant la séquence d'acide nucléique selon l'une des revendications 20 et 21.

24. Séquence d'acide nucléique comportant le plasmide pGN comme illustré dans la figure 1, et de part et d'autre de la séquence pGN deux séquences flanquantes respectivement homologues aux deux séquences génomiques, qui jouxtent un site d'insertion souhaité dans un gène receveur.

25. Cellules eucaryotes transformées par le procédé de la revendication 1, 2 ou 7.

26. Cellules selon la revendication 25 caractérisées en ce que ce sont des cellules E.S.

27. Utilisation de la séquence d'acide nucléique selon la revendication 20 pour la modification de cellules eucaryotes en vue de rendre l'expression de ladite séquence, ou d'une séquence endogène, fonctionnelle ou plus efficace.

28. Séquence d'acide nucléique comprenant :
i) une séquence régulatrice destinée à être recombinée avec un ADN de complément pour alors fournir un gène recombinant complet dans le génome d'une cellule eucaryote, et
ii) de part et d'autre de la séquence i) deux séquences dites "flanquantes" respectivement homologues aux deux séquences génomiques qui jouxtent le site d'insertion souhaité dans un gène receveur.

## Claims

1. Method for the specific replacement or insertion of a gene, in particular by targeting a DNA, termed insertion DNA, consisting of the whole of a gene or of a portion of a gene capable of being rendered functional, or whose functioning may be rendered more effective, when it is recombined with a complementary DNA to yield then a complete recombinant gene in the genome of a eukaryotic cell, characterized in that
- the insertion site is in a chosen gene, termed chosen recipient gene, containing the complementary DNA, and in that
- eukaryotic cells are transfected with a vector containing an insert itself comprising the insertion DNA and two so-called "flanking" sequences on each side of the insertion DNA, which are homologous, respectively, to two genomic sequences which adjoin the desired insertion site in the recipient gene,
- the insertion DNA being heterologous with respect to the recipient gene, and
- the flanking sequences being chosen from those which constitute the abovementioned complementary DNA and which permit, by homologous recombination with corresponding sequences of the recipient gene, the reconstitution of a complete recombinant gene in the genome of the eukaryotic cell, on condition that, when the insertion DNA consists of a coding sequence, it is not the coding sequence of a gene coding for a selectable agent.

2. Method for the specific replacement or insertion of a gene, in particular by targeting a DNA, termed insertion DNA, consisting of the whole of a gene or of a portion of a gene capable of being rendered functional, or whose functioning may be rendered more effective, when it is recombined with a complementary DNA to yield then a complete recombinant gene in the genome of a eukaryotic cell, characterized in that
- the insertion site is in a chosen gene, termed chosen recipient gene, containing the complementary DNA, and in that
- eukaryotic cells are transfected with a vector containing an insert itself comprising the insertion DNA and two so-called "flanking" sequences on each side of the insertion DNA, which are homologous, respectively, to two genomic sequences which adjoin the desired insertion site in the recipient gene,
- the insertion DNA being heterologous with respect to the recipient gene, and
- the flanking sequences being chosen from those which constitute the abovementioned complementary DNA and which permit, by homologous recombination with corresponding sequences of the recipient gene, the reconstitution of a complete recombinant gene in the genome of the eukaryotic cell,
- the recipient gene being a gene which is not normally transcribed in the eukaryotic cell.

3. Method according to Claim 1 or 2, characterized in that the said insertion DNA contains either a coding sequence or a regulatory sequence,
- the flanking sequences being chosen so as to permit, by homologous recombination, depending on the case, either the expression of the coding sequence of the entire insertion DNA under the control of the regulatory sequences of the recipient gene, or the expression of a coding sequence of the recipient gene under the control of regulatory sequences of the insertion DNA.

4. Method according to any of Claims 1, 2 and 3, characterized in that the insertion DNA contains a coding sequence lacking a regulatory element, in particular a promoter which is distinctive to it.

5. Method according to Claim 3, characterized in that the insertion DNA contains a regulatory sequence.

6. Method according to Claim 5, characterized in that the endogenous promoter of the recipient gene is inactive in the transfected eukaryotic cells.

7. Method for the specific replacement of a regulatory sequence of a gene, termed recipient gene, by targeting a DNA, termed insertion DNA, the said insertion DNA containing a regulatory sequence, characterized in that
- eukaryotic cells are transfected with a vector containing an insert itself comprising the insertion DNA and two so-called "flanking" sequences on each side of the insertion DNA, which are homologous, respectively, to two genomic sequences which adjoin the desired insertion site in the recipient gene,
- the insertion DNA being heterologous with respect to the recipient gene, and
- the flanking sequences being chosen so as to permit, by homologous recombination, the expression of a coding sequence of the recipient gene under the control of regulatory sequences of the insertion DNA.

8. Method according to Claim 7, characterized in that the endogenous promoter of the recipient gene is normally inactive in the transfected eukaryotic cells.

9. Method according to any one of the preceding claims, characterized in that the recipient gene is present in the genome of the eukaryotic cell in at least two copies.

10. Method according to any one of the preceding claims, characterized in that each of the flanking sequences has a length greater than 150 base pairs, and smaller than the length of the recipient gene.

11. Method according to any one of Claims 1 to 10, characterized in that the eukaryotic cells are embryonic stem (ES) cells.

12. Method according to one of the preceding claims, characterized in that the insertion DNA is a gene which is heterologous or a portion of a gene which is heterologous with respect to the transfected species.

13. Method according to one of the preceding claims, characterized in that the vector contains sequences interposed between the insertion gene and the flanking sequences.

14. Method according to Claim 13, characterized in that the interposing sequences contain a sequence coding for a selective agent permitting selection of the transformants and, where appropriate, a marker gene, for example the lacZ gene.

15. Method according to one of the preceding claims, characterized in that the transfection is performed by electroporation.

16. Method according to one of the preceding claims, characterized in that the polymerase chain reaction (PCR) technique is used to amplify the DNA sequence of the locus where the insertion takes place in order to verify that the insertion has taken place in the desired site.

17. Method according to any one of the preceding claims, characterized in that the insertion DNA contains a portion of the interferon or interleukin gene, of genes for receptors such as the β₃-adrenergic retinoic acid receptor or the HIV receptor, or of a gene linked to a disorder, or of the lacZ gene.

18. Method according to any one of the preceding claims, characterized in that the insertion DNA contains, between the flanking sequences, on the one hand a DNA sequence intended to be recombined with the complementary DNA in the recipient gene to yield a recombinant gene, and on the other hand a sequence coding for a selective agent permitting selection of the transformants and a promoter permitting expression of the selective agent.

19. Method for the production of transgenic animals, characterized in that ES cells are transfected by the method according to one of Claims 1 to 18 and screened for the homologous recombination event, namely the correct integration of the foreign gene, the cells are injected into embryos at a stage at which they are capable of integrating the transfected cells, for example at the blastocyst stage, the embryos are then reimplanted in a surrogate mother, and the chimeric individuals obtained at the end of gestation, and in which colonization by ES cells of the germ line is observed, are mated to obtain transgenic animals which are heterozygous for the replaced gene.

20. Nucleic acid sequence, termed insertion sequence, comprising:
i) a sequence comprising all or part of a gene capable of being rendered functional, or whose functioning may be rendered more effective, when it is recombined with a complementary DNA to yield then a complete recombinant gene in the genome of a eukaryotic cell, on condition that, when the portion of the gene consists of a coding sequence, it is a coding sequence other than that of a gene coding for a selectable agent,
ii) on each side of the sequence i), two so-called "flanking" sequences homologous, respectively, to the two genomic sequences which adjoin the desired insertion site in the recipient gene.

21. Nucleic acid sequence according to Claim 20, characterized in that it comprises, in addition, between the flanking sequences, a sequence coding for a selective agent permitting selection of the transformants and a promoter permitting expression of the selective agent.

22. Nucleic acid sequence according to Claim 20 or 21, characterized in that it comprises, in addition, a sequence removed by the homologous recombination process, and enabling the transformants originating from homologous recombination events to be selected.

23. Vector containing the nucleic acid sequence according to either of Claims 20 and 21.

24. Nucleic acid sequence containing plasmid pGN as illustrated in Figure 1 and, on each side of the pGN sequence, two flanking sequences homologous, respectively, to the two genomic sequences which adjoin a desired insertion site in a recipient gene.

25. Eukaryotic cells transformed by the method of Claim 1, 2 or 7.

26. Cells according to Claim 25, characterized in that they are ES cells.

27. Use of the nucleic acid sequence according to Claim 20 for the modification of eukaryotic cells for the purpose of rendering the expression of the said sequence, or of an endogenous sequence, functional or more effective.

28. Nucleic acid sequence comprising:
i) a regulatory sequence intended to be recombined with a complementary DNA to yield then a complete recombinant gene in the genome of a eukaryotic cell, and
ii) on each side of the sequence i), two so-called "flanking" sequences homologous, respectively, to the two genomic sequences which adjoin the desired insertion site in a recipient gene.

## Patentansprüche

1. Verfahren zum spezifischen Austausch oder zur spezifischen Insertion eines Gens, insbesondere durch gezieltes Einführen einer DNA, wobei die DNA-Insertion aus der Gesamtheit eines Gens oder aus einem Teil eines Gens besteht, das in eine funktionelle Form überführt werden kann oder dessen Funktionalität gesteigert worden kann, wenn es mit einer komplementären DNA rekombiniert wird, um so in dem Genom einer eukaryontischen Zelle ein vollständiges rekombinantes Gen zu bilden, dadurch gekennzeichnet, daß
- sich der Ort der Insertion in einem ausgewählten Gen, dem ausgewählten Empfängergen, befindet, und die komplementäre DNA enthält, und dadurch daß
- eukaryontische Zellen mit einem Vektor transfiziert werden, der eine Insertion enthält, die wiederum die DNA-Insertion umfaßt und zwei sogenannte "flankierende" Sequenzen beiderseits der DNA-Insertion, die jeweils homolog sind zu zwei genomischen Sequenzen, die neben der gewünschten Insertionsstelle in dem Empfängergen liegen,
- die DNA-Insertion in bezug auf das Empfängergen heterolog ist, und
- die flankierenden Sequenzen aus solchen ausgewählt werden, die die obengenannte komplementäre DNA darstellen und die über eine homologe Rekombination mit korrespondierenden Sequenzen des Empfängergens die Rekonstitution eines vollständigen rekombinanten Gens in dem Genom der eukaryontischen Zelle ermöglichen, unter der Bedingung, daß, wenn die DNA-Insertion von einer codierenden Sequenz gebildet wird, es sich nicht um die codierende Sequenz eines Gens handelt, das einen Selektionsmarker codiert.

2. Verfahren zum spezifischen Austausch oder zur spezifischen Insertion eines Gens, insbesondere durch gezieltes Einführen einer DNA, wobei die DNA-Insertion aus der Gesamtheit eines Gene oder aus einem Teil eines Gens besteht, das in eine funktionelle Form überführt werden kann oder dessen Funktionalität gesteigert werden kann, wenn es mit einer komplementären DNA rekombiniert wird, um so in dem Genom einer eukaryontischen Zelle ein vollständiges rekombinantes Gen zu bilden, dadurch gekennzeichnet, daß
- sich der Ort der Insertion in einem ausgewählten Gen, dem ausgewählten Empfängergen, befindet, und die komplementäre DNA enthält, und dadurch, daß
- eukaryontische Zellen mit einem Vektor transfiziert werden, der eine Insertion enthält, die wiederum die DNA-Insertion umfaßt und zwei sogenannte "flankierende" Sequenzen beiderseits der DNA-Insertion, die jeweils homolog sind zu zwei genomischen Sequenzen, die neben der gewünschten Insertionsstelle in dem Empfängergen liegen,
- die DNA-Insertion in bezug auf das Empfängergen heterolog ist, und
- die flankierenden Sequenzen aus solchen ausgewählt sind, die die obengenannte komplementäre DNA darstellen und die über eine homologe Rekombination mit korrespondierenden Sequenzen des Empfängergens die Rekonstitution eines vollständigen rekombinanten Gens in dem Genom der eukaryontischen Zelle ermöglichen,
- das Empfängergen ein Gen ist, das normalerweise in der eukaryontischen Zelle nicht transkribiert wird.

3. Verfahren nach Anspruch 1 oder 2, dadurch gekennzeichnet, daß die DNA-Insertion entweder eine codierende Sequenz oder eine regulatorische Sequenz umfaßt,
- die flankierenden Sequenzen derart gewählt sind, daß sie aufgrund homologer Rekombination je nachdem entweder die Expression der codierenden Sequenz der gesamten DNA-Insertion unter der Kontrolle von regulatorischen Sequenzen des Empfängergens erlauben oder die Expression einer codierenden Sequenz des Empfängergens unter dar Kontrolle der regulatorischen Sequenzen der DNA-Insertion.

4. Verfahren nach einem der Ansprüche 1, 2 oder 3, dadurch gekennzeichnet, daß die DNA-Insertion eine codierende Sequenz umfaßt, die keine regulatorischen Elemente aufweist, insbesondere keinen eigenen Promotor.

5. Verfahren nach Anspruch 3, dadurch gekennzeichnet, daß die DNA-Insertion eine regulatorische Sequenz umfaßt.

6. Verfahren nach Anspruch 5, dadurch gekennzeichnet, daß der endogene Promotor des Empfängergens in den transfizierten eukaryontischen Zellen inaktiv ist.

7. Verfahren zum spezifischen Austausch einer regulatorischen Sequenz eines Gens, des Empfängergens, durch gezieltes Einführen einer DNA, der DNA-Insertion, wobei die DNA-Insertion eine regulatorische Sequenz enthält, dadurch gekennzeichnet, daß
- eukaryontische Zellen mit einem Vektor transfiziert werden, der eine Insertion enthält, die wiederum die DNA-Insertion umfaßt und zwei sogenannte "flankierende" Sequenzen beiderseits der DNA-Insertion, die jeweils homolog sind zu zwei genomischen Sequenzen, die neben der gewünschten Insertionsstelle in dem Empfängergen liegen,
- die DNA-Insertion in bezug auf das Empfängergen heterolog ist, und
- die flankierenden Sequenzen derart gewählt sind, daß sie aufgrund homologer Rekombination die Expression einer codierenden Sequenz des Empfängergens unter der Kontrolle der regulatorischen Sequenzen der DNA-Insertion erlauben.

8. Verfahren nach Anspruch 7, dadurch gekennzeichnet, daß der endogene Promotor des Empfängergens normalerweise in den transfizierten eukaryontischen Zellen inaktiv ist.

9. Verfahren nach einem der vorangehenden Ansprüche, dadurch gekennzeichnet, daß das Empfängergen in dem Genom der eukaryontischen Zelle in mindestens zwei Kopien vorkommt.

10. Verfahren nach einem der vorangehenden Ansprüche, da durch gekennzeichnet, daß jede der flankierenden Sequenzen länger als 150 Basenpaare und kürzer als das Empfängergen ist.

11. Verfahren nach einem der Ansprüche 1 bis 10, dadurch gekennzeichnet, daß die eukaryontischen Zellen embryonale Stammzellen (E.S.) sind.

12. Verfahren nach einem der Ansprüche 1 bis 11, dadurch gekennzeichnet, daß die DNA-Insertion ein heterologes Gen oder ein Teil eines heterologen Gens ist in bezug auf die transfizierte Spezies.

13. Verfahren nach einem der vorangehenden Ansprüche, dadurch gekennzeichnet, daß der Vektor zwischen dem Gen der Insertion und den flankierenden Sequenzen intervenierende Sequenzen enthält.

14. Verfahren nach Anspruch 13, dadurch gekennzeichnet, daß die intervenierenden Sequenzen eine Sequenz umfassen, die einen Selektionsmarker codiert, der die Selektion der Transformanten erlaubt, und gegebenenfalls ein Markergen, wie z.B. das lacZ-Gen.

15. Verfahren nach einem der vorangehenden Ansprüche, dadurch gekennzeichnet, daß die Transfektion mittels Elektroporation durchgeführt wird.

16. Verfahren nach einem der vorangehenden Ansprüche, dadurch gekennzeichnet, daß die Polymerasekettenreaktion (P.C.R.) verwendet wird für die Amplifikation der DNA-Sequenz des Locus, an dem sich die Insertion befindet, um nachzuprüfen, ob die Insertion an der gewünschten Stelle stattgefunden hat.

17. Verfahren nach einem der vorangehenden Ansprüche, dadurch gekennzeichnet, daß die DNA-Insertion einen Teil eines Interferongens, eines Interleukingens, eines Rezeptorgens, wie z.B. des Retinsäure-, des β-3-adrenergen oder des HIV-Rezeptors, oder eines mit einer Krankheit in Verbindung stehenden Gens oder des lacZ-Gens umfaßt.

18. Verfahren nach einem der vorangehenden Ansprüche, dadurch gekennzeichnet, daß die DNA-Insertion zwischen den flankierenden Sequenzen einerseits eine DNA-Sequenz enthält, die dazu bestimmt ist, mit der komplementären DNA in dem Empfängergen rekombiniert zu werden, um ein rekombinantes Gen zu erzeugen, und andererseits eine einen Selektionsmarker codierende Sequenz, der die Selektion von Transformanten erlaubt, und einen Promotor, der die Expression des Selektionsmarkers kontrolliert.

19. Verfahren zur Herstellung transgener Tiere, dadurch gekennzeichnet, daß E.S.-Zellen gemäß dem Verfahren nach einem der Ansprüche 1 bis 18 transfiziert werden und durchgemustert werden hinsichtlich des Auftretens einer homologen Rekombination, um die korrekte Integration des Fremdgens sicherzustellen, die Zellen in Embryonen injiziert werden, die sich in einem Stadium befinden, in dem sie in der Lage sind, die transfizierten Zellen zu integrieren, z.B. im Blastozysten-Stadium, und welche anschließend in eine Leihmutter reimplantiert werden, und die chimären Individuen, die am Ende der Schwangerschaft erhalten werden und bei denen die Kolonisierung durch E.S.-Zellen, die aus dem Keim stammen, festgestellt wird, gekreuzt werden, um transgene Tiere zu erhalten, die hinsichtlich des ausgetauschten Gens heterozygot sind.

20. Nucleinsäuresequenz, nämlich die Insertionssequenz, enthaltend:
i) eine Sequenz, die die Gesamtheit oder einen Teil eines Gens umfaßt, das in eine funktionsfähige Form überführt werden kann oder dessen Funktionalität gesteigert werden kann, wenn es mit einer komplementären DNA rekombiniert wird, um so in dem Genom einer eukaryontischen Zelle ein vollständiges rekombinantes Gen zu bilden, unter der Bedingung, daß, wenn der Teil des Gens aus einer codierenden Sequenz besteht, es sich um eine codierende Sequenz handelt, die nicht von einem Gen stammt, das einen Selektionsmarker codiert,
ii) zwei sogenannte "flankierende" Sequenzen beiderseits der unter i) genannten Sequenz, die jeweils homolog sind zu zwei genomischen Sequenzen, die neben der gewünschten Insertionsstelle in dem Empfängergen liegen.

21. Nucleinsäuresequenz nach Anspruch 20, dadurch gekennzeichnet, daß sie unter anderem zwischen den flankierenden Sequenzen eine einen Selektionsmarker, der die Selektion von Transformanten erlaubt, codierende Sequenz enthält und einen Promotor, der die Expression des Selektionsmarkers kontrolliert.

22. Nucleinsäuresequenz nach Anspruch 20 oder 21, dadurch gekennzeichnet, daß sie unter anderem eine Sequenz enthält, die durch den Vorgang einer homologen Rekombination eliminiert wird und die die Selektion von Transformanten erlaubt, die durch das Ereignis einer homologen Rekombination entstanden sind.

23. Vektor, enthaltend die Nucleinsäuresequenz nach Anspruch 20 oder 21.

24. Nucleinsäuresequenz enthaltend das Plasmid pGN, wie in Figur 1 dargestellt, und beiderseits der pGN-Sequenz zwei flankierende Sequenzen, die jeweils homolog sind zu zwei genomischen Sequenzen, die neben einer gewünschten Insertionsstelle in einem Empfängergen liegen.

25. Eukaryontische Zellen, die gemäß dem Verfahren nach Anspruch 1, 2 oder 7 transformiert wurden.

26. Zellen nach Anspruch 25, dadurch gekennzeichnet, daß es sich um E.S.-Zellen handelt.

27. Verwendung der Nucleinsäuresequenz nach Anspruch 20 für die Modifikation eukaryontischer Zellen im Hinblick auf eine funktionelle oder effizientere Expression der Sequenz oder einer endogenen Sequenz.

28. Nucleinsäuresequenz umfassend:
i) eine regulatorische Sequenz, die dazu vorgesehen ist, mit einer komplementären DNA rekombiniert zu werden, um so in dem Genom einer eukaryontischen Zelle ein vollständiges rekombinantes Gen zu erzeugen, und
ii) beiderseits der unter i) genannten Sequenz zwei sogenannte "flankierende" Sequenzen, die jeweils homolog sind zu zwei genomischen Sequenzen, die neben der gewünschten Insertionsstelle in einem Empfängergen liegen.
